# EUROPEAN PATENT SPECIFICATION

(11) **EP 2 052 033 B1**
(45) Date of publication and mention of the grant of the patent: **04.05.2011**
(21) Application number: 07807593.4
(22) Date of filing: 12.09.2007
(51) Int. Cl.: C07D 403/08, C09B 57/00, G11B 7/246, G11B 7/247, G11B 7/249

(54) **SQUARYLIUM COMPOUND, OPTICAL RECORDING MEDIUM USING THE SAME, RECORDING/REPRODUCTION METHOD AND OPTICAL RECORDING APPARATUS**
SQUARYLIUMVERBINDUNG, DAVON GEBRAUCH MACHENDES OPTISCHES AUFZEICHNUNGSMEDIUM, AUFZEICHNUNGS-/WIEDERGABEVERFAHREN UND OPTISCHES AUFZEICHNUNGSGERÄT
COMPOSÉ À BASE DE SQUARYLIUM, SUPPORT D'ENREGISTREMENT OPTIQUE UTILISANT UN TEL COMPOSÉ, PROCÉDÉ D'ENREGISTREMENT/DE REPRODUCTION ET APPAREIL D'ENREGISTREMENT OPTIQUE

(30) Priority: 19.09.2006 JP 2006253184
(43) Date of publication of application: 29.04.2009
(73) Proprietor: Ricoh Company, Ltd., Tokyo 143-8555 (JP); Kyowa Hakko Chemical Co., Ltd., Tokyo 103-0022 (JP)
(72) Inventor: YASHIRO, Tohru, Yokosuka-shi, Kanagawa 239-0807 (JP); ISHIMI, Tomomi, Shizuoka 410-0004 (JP); KINUGASA, Motoharu, Mie 510-0022 (JP)
(74) Representative: Barz, Peter
(86) International application number: PCT/JP2007/068225
(87) International publication number: WO 2008/035724

(56) References cited:
- EP-A- 1 505 125
- JP-A- 2002 370 451
- JP-A- 2006 063 221
- JP-A- 2006 150 855
- JP-A- 2006 150 856

## Description

### Technical Field

The present invention relates to a squarylium compound forming a metal complex that can be used in the field of optical recording, an optical recording medium using the same, a recording/reproduction method and an optical recording apparatus.

### Background Art

Recordable DVDs (DVD+RW, DVD+R, DVD-R, DVD-RW, DVD-RAM, etc.) have become commercially available in addition to optical recording mediums such as a DVD-ROM (see, inter alia, Patent Documents 1, 2 and 3).

Of the above, a DVD+R and a DVD+RW are technologically on the line of a CD-R (compact disk-recordable) and a CD-RW (compact disk-rewritable) and hence they are designed to meet the requirements of a CD and also those of a DVD in terms of recording density (track pitch and signal mark length) and substrate thickness in order to make them compatible with a DVD-ROM.

For instance, a DVD+R has a recording layer on a substrate and an information recording substrate carrying a reflection layer is bonded onto the recording layer on the substrate, the two substrates having the same profile. A dye type material is used for the recording layer.

One of the characteristics of a CD-R is that it has a high reflectance (65%) to meet the requirements of the CD standards. To realize such a high reflectance with the above-described arrangement, the recording layer needs to show a specific complex refractive index for the wavelength of light for recording/reproduction. To meet this requirement, organic dye materials are more suited than inorganic materials from the viewpoint of light absorption characteristics. DVDs are also required to this requirement.

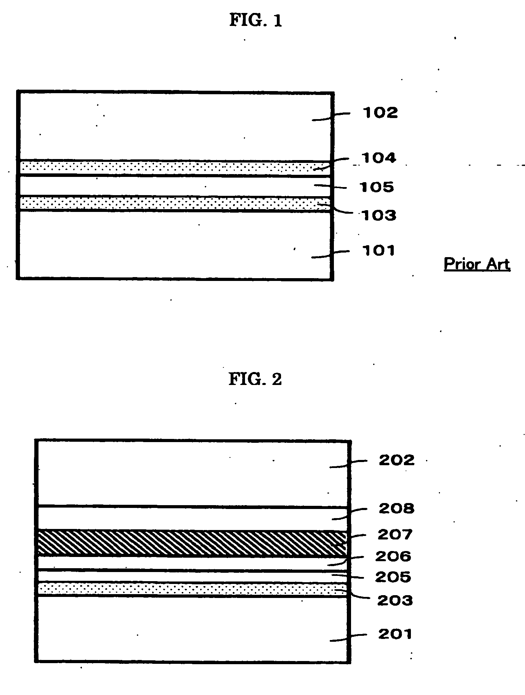

As for DVD-ROMs, DVDs having two recording layers have been proposed in order to increase the recording capacity of the disk. For example, FIG. 1 of the accompanying drawings is a schematic cross sectional view of a known optical recording medium (a DVD having two recording layers). Referring to FIG. 1, a first substrate 101 and a second substrate 102 are bonded to each other with an intermediate layer 105 that is made of ultraviolet curing resin interposed between them. A first recording layer 103 is formed on the inner surface of the first substrate 101, while a second recording layer 104 is formed on the inner surface of the second substrate 102. The first recording layer 103 is realized as a translucent film that is typically a dielectric film. The second recording layer 104 is realized as a reflective film that is typically a metal film. Recording marks are formed on the first recording layer 103 as undulations so that the recorded signals are read out by means of the effect of reflecting and interfering with a reproduction laser beam. The DVD illustrated in FIG. 1 can maximally provide a storage capacity of about 8.5 GB because signals are read out from the two recording layers.

Each of the first substrate 101 and the second substrate 102 has a thickness of about 0.6 mm and the intermediate layer 105 has a thickness of about 50 µm. The translucent film of the first recording layer 103 is made to show a reflectance of about 30% so that the laser beam irradiated to reproduce signals from the second recording layer 104 is reflected by the first recording layer 103 by about 30% and attenuated in terms of total quantity of light. Subsequently, the laser beam is reflected by the reflective film of the second recording layer 104 and attenuated further by the first recording layer 103 before it is emitted from the optical recording medium. The laser beam that is reproducing light is converged so as to be focused on the first recording layer or the second recording layer and the signals on the recording layers can be reproduced by detecting the reflected light. In the case of DVDs, the wavelength of the laser beam to be used for signal recording/reproduction is about 650 nm.

Similarly, a DVD+R and a DVD-R are being discussed as optical recording mediums having dual layers for signal recording/reproduction on one of the surfaces thereof. Such a disk is described in the Patent Document 1 for instance.

However, as a result of the development of high-speed recording systems in recent years, there is a demand for disks that are adapted to record signals at a high linear velocity. More specifically, while a recording/reproduction standard linear velocity of 3.49 m/s is defined for DVDs having single layer as a recording layer on one of the surfaces thereof and that of 3.83 m/s is defined for DVDs having dual layer as a recording layer on one of the surfaces thereof, a recording speed that is about 12 times to 18 times as high as those standard linear velocities is required for such disks.

It is difficult for conventional recording mediums to meet such a high-speed requirement. More specifically, there would arise a problem that degradation of signal jitter occurs due to thermal interference in the formation of the recorded marks.

In short, one-side double-layer recording mediums including the one disclosed in the above Patent Document 1 are accompanied by a problem that it is not possible to achieve a sufficient recording speed and a satisfactory recording power if compared with conventional one-side single-layer recording mediums having a single recording layer on one of the surfaces thereof.

The reason for this problem lies in that a one-side double-layer recording medium has two recording layers on one of the surfaces thereof by definition and hence, when a signal writing laser beam is emitted from an optical pickup and focused on the deeper recording layer to record signals, the first recording layer attenuates the laser beam to make it difficult to compatibly give rise to absorption and reflection of light to a level necessary for recording on the second recording layer.

Particularly, the second recording layer is accompanied by the problem of difficulty of forming recording marks because the recording layer has a configuration different from the recording layers of existing CD-R and DVD+R. This is because substrate/dye layer/reflective layer/protection layer are formed in the above-mentioned order from the light entering side of existing recording/reproduction mediums having a recording layer, whereas bonding layer/(inorganic protection layer)/recording layer/reflective layer/substrate are formed in the above-mentioned order from the light receiving side for the second recording layer of recording/reproduction mediums having two recording layers. This means that the recording mark forming environment (adjacent layers) of the recording layers of the double-layer recording medium differs from that of the single-layer recording medium. Then, it is difficult to control the formation of recording marks when the recording linear velocity is high to consequently give rise to a problem of difficulty of achieving recording/reproduction characteristics adapted to DVD systems in terms of modulation and jitter.

Meanwhile, cyanine dyes, azo dyes and squarylium dyes are being put to practical use as recording layer dye materials for DVDs. However, these organic dye materials are poorly resistive against light so that, when a recording layer is formed by means of a cyanine dye or a squarylium dye, a mixture of the dye and a light resisting agent, which may be an aluminum compound, a bis-thiol metal complex, a formazan metal chelate compound or an azo metal chelate compound is used to provide resistance against light to a practically feasible level

For example, the applicant of the present patent application has proposed techniques of mixing a squarylium metal chelate compound showing excellent disk recording characteristics and disclosed in the Patent Documents 2 and 3 with any of various light resisting agents such as a formazan metal chelate compound (see Patent Documents 4, 5 and 6) or an azo metal chelate compound (see Patent Documents 7 and 8) in order to provide a satisfactory level of resistance against light for the recording layer.

However, when a squarylium metal chelate compound is mixed with a light resisting agent, the optical characteristics and the thermal decomposition characteristics of the film are changed to such an extent that the recording/reproduction performance of the recording layer is degraded if the resistance against light is made practically feasible.

There have been proposed a squarylium compound having a specific structure and adapted to be used for an optical recording medium that can record information by means of irradiation of light and an optical recording medium having a recording layer containing such a compound (see Patent Document 9). The Patent Document claims that jitters are reduced with such an optical recording medium and the recording medium additionally shows an improved resistance against light and weather. Examples 1 through 15 in the above-cited Patent Document describe the results of preparing optical recording disks having a recording layer formed by solely using a squarylium compound and conducting tests on the resistance against light and tests on the effects of storage. However, the Examples do not describe any salt or chelate compound formed by a squarylium compound and metal and the use.

A dye of a salt of a squarylium compound having a specific structure to be used for an optical recording medium adapted to record information by means of irradiation of light and an optical recording medium having a recording layer containing such a dye have been proposed (see Patent Document 10). The Patent Document claims that such an optical recording medium shows improvements in terms of resistance against light, reliability and reflectance and reduces jitters. Examples 1 through 3 in the above-cited Patent Document describe the results of preparing optical recording disks having a recording layer formed by solely using a compound expressed by the general formula (I), where each of R³ and R⁴ represents a methyl group, and conducting tests on the resistance against light and tests on the effects of storage.

While the above-cited proposal may provide certain improvements of characteristics at the linear velocity of 3.5 m/s for signal reproduction as described in the Examples, it may be difficult to achieve satisfactory reproduction characteristics at a higher linear velocity (e.g., 3.83 m/s).

As described above, there is an urgent demand for developing a novel recording/reproducing type optical recording medium having two recording layers on one side thereof, which attains both excellent signal characteristics and excellent light resistance at the recording speed 12 times to 18 times faster than the standard recording speed, has a first recording layer and a second recording layer as the two recording layers, and exhibits both excellent recording signal characteristics and excellent light resistance in the second recording layer located at the side of a second substrate.
[Patent Document 1] Japanese Patent Application Laid-Open (JP-A) No. 2006-44241
[Patent Document 2] International Publication No. 01/44233 Pamphlet
[Patent Document 3] International Publication No. 01/44375 Pamphlet
[Patent Document 4] Japanese Patent Application Laid-Open (JP-A) No. 2002-370451
[Patent Document 5] Japanese Patent Application Laid-Open (JP-A) No. 2002-370452
[Patent Document 6] Japanese Patent Application Laid-Open (JP-A) No. 2004-244342
[Patent Document 7] Japanese Patent Application Laid-Open (JP-A) No. 2002-370453
[Patent Document 8] Japanese Patent Application Laid-Open (JP-A) No. 2002-370454
[Patent Document 9] Japanese Patent Application Laid-Open (JP-A) No. 2006-150855
[Patent Document 10] Japanese Patent Application Laid-Open (JP-A) No. 2006-150856

### Disclosure of the Invention

In view of the above identified circumstances, it is therefore the challenge to the present invention to dissolve the above-listed problems and achieve the following object.

The object of the present invention is to provide a squarylium compound forming a metal complex that shows good signal recording characteristics at high-speed recording by irradiation of light and an excellent resistance against light (to be referred to as a squarylium metal chelate compound hereinafter), a one-side single-layer recording medium or a one-side double-layer recording medium (having a first recording layer and a second recording layer) containing a squarylium metal chelate compound on the recording layer, a recording/reproduction method using such an optical recording medium and an optical recording apparatus.

The inventors of the present invention found that it is possible to obtain an optical recording medium compatibly showing both optical characteristics useful for a DVD optical recording system using a laser of the 645 nm to 675 nm band and an improved resistance against light by using a squarylium metal chelate compound having a specific compound as a dye for the optical recording layers and achieved the present invention. Thus, the above-listed problems are solved by the present invention described in claims 1-12.

A squarylium compound forming a metal complex (squarylium metal chelate compound) according to the present invention has a specific structure expressed by the above general formula (I), shows good recording characteristics in a recording wavelength (typically from 580 nm to 720 nm) by controlling its optical characteristics, and an improved resistance against light. Then, there are provided optical recording mediums adapted to DVD disk systems including so called optical recording medium having a recording layer on one of the surfaces thereof and optical recording medium having two recording layers on one of the surfaces thereof.

An optical recording medium according to the present invention contains a squarylium metal chelate compound in the recording layer thereof and hence it shows good recording signal characteristics at high-speed recording using irradiation of light and operates excellently without any degradation of the resistance of the recording layer against repeated irradiation of light for signal recording/reproduction and exposure to sun light. Therefore, an optical recording medium according to the present invention can suitably be used for a recordable DVD system. Particularly, an optical recording medium according to the present invention can broadly find applications including DVD+R and DVD-R. More specifically, an optical recording medium according to the present invention satisfies the requirements of the standards for DVDs in terms of stabilized reflectance, high percentage modulation, jitter and PI error (Pisum8).

Additionally, the squarylium metal chelate compound may be mixed with some other light resisting agent (such as a formazan metal chelate compound) for the recording layer to secure the recording/reproduction performance and the practically feasible resistance against light of the recording layer in a well balanced manner.

A recording/reproduction method according to the present invention employs a recording medium having a recording layer that is made of a squarylium metal chelate compound so that it is not accompanied by any problem such as broadened recording marks, cross talks to adjacent tracks and increased jitters. Therefore, the recording medium can show good recording characteristics typically with a recording wavelength from 580 nm to 720 nm and operate stably if repeatedly irradiated with light.

An optical recording apparatus according to the present invention is mounted by an optical recording medium having a recording layer that is made of a squarylium metal chelate compound and a means for recording information on and reproducing information from the optical recording memory by irradiating the recording medium with light from a light source so that it is possible to record information on and reproduce information from a large capacity DVD system at high-speed.

### Brief Description of the Drawings

FIG. 1 is a schematic cross sectional view of a conventional optical recording medium, showing the layer arrangement thereof.
FIG. 2 is a schematic cross sectional view of the first embodiment of an optical recording medium according to the present invention, showing the layer arrangement thereof.
FIG. 3 is a schematic cross sectional view of the second embodiment of an optical recording medium according to the present invention, showing the layer arrangement thereof.
FIG. 4 is a schematic cross sectional view of the first information layer of a one-side double-layer recording medium according to the present invention, showing the configuration thereof.
FIG. 5 is a schematic cross sectional view of the second information layer of a one-side double-layer recording medium according to the present invention, showing the configuration thereof.
FIG. 6 is a graph illustrating the relationship between a desired absorption spectrum of the dye to be used in a recording layer and the recording and reproduction wavelength.
FIG. 7 is a graph illustrating the signal evaluation results (the recording power dependency of jitters in 16x recording) of Example 17.
FIG. 8 is a graph illustrating the signal evaluation results (the recording power dependency of jitters in 18x recording) of Example 18.

### Best Mode for Carrying Out the Invention

As described above, a squarylium compound froming a metal complex (squarylium metal chelate compound) according to the present invention shows good signal recording characteristics at high-speed recording by irradiation of light and an excellent resistance against light and is characterized by being expressed by the general formula (I) below:

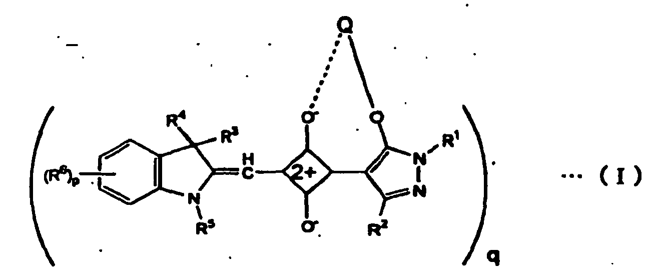

In the general formula (I), R¹ to R⁶, Q, p and q are as defined in claim 1.

In short, a squarylium metal chelate compound according to the present invention can suitably be applied to a one-side single-layer recording medium or a one-side double-layer recording medium (having a first recording layer and a second recording layer) as dye material for the recording layer or layers.

Additionally, an optical recording medium according to the present invention shows good optical characteristics (including a high reflectance) and good recording signal characteristics (in terms of jitter and PI error) so that it is possible to provide a recording/reproduction method and an optical recording apparatus that are highly reliable by using an optical recording medium according to the present invention. Note that a PI error is a sum of the PI errors in every consecutive 8 ECC blocks.

A one-side single-layer recording medium and a one-side double-layer recording medium according to the present invention will be described in greater detail hereinafter.

Examples of metal atoms M having a coordinating ability include aluminum, zinc, copper, iron, nickel, chromium, cobalt, manganese, iridium, vanadium and titanium.

Particularly, a squarylium compound forming an aluminum complex is preferable because it shows excellent optical characteristics.

Of the above, a squarylium compound forming an aluminum complex and having a benzyl group at the third position of the indolinium group is particularly preferable because the compound can minimize cross talks.

In an optical recording medium according to the present invention, the recording layer made of a squarylium metal chelate compound according to the present invention preferably contains a squarylium compound forming a metal complex and expressed by the general formula (II) below because it leads to more stable recording characteristics.

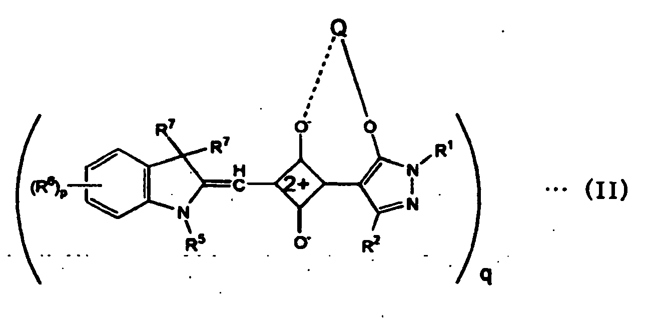

In the general formula (II), where R¹ and R² are the same or different to each other and each of R¹ and R² represents a substituted or unsubstituted alkyl group, a substituted or unsubstituted aralkyl group, a substituted or unsubstituted aryl group, or a substituted or unsubstituted heterocyclic group; Q represents a metal atom having coordinating ability; q represents 2 or 3; R⁷ represents a substituted or unsubstituted alkyl group, or two R⁷s are bonded to each other to form either an alicyclic hydrocarbon ring or heterocyclic ring; R⁵ represents a hydrogen atom, a substituted or unsubstituted alkyl group, a substituted or unsubstituted aralkyl group, or a substituted or unsubstituted aryl group; R⁶ represents a halogen atom, a substituted or unsubstituted alkyl group, a substituted or unsubstituted aralkyl group, a substituted or unsubstituted aryl group, a nitro group, a cyano group, or a substituted or unsubstituted alkoxy group; p represents any integer ranging from 0 to 4; and R⁶s are the same or different to each other and any two adjacently located R⁶s form a substituted or unsubstituted aromatic ring together with two adjacent carbon atoms when p is 2, 3 or 4.

As for the definition of a substituent in the structural formulas (I) and (II), the alkyl part in an alkyl group and an alkoxy group may be an alkyl group having 1 carbon atom to 6 straight chain or branched chain carbon atoms or a cyclic alkyl group having 3 carbon atoms to 8 carbon atoms. Examples of alkyl groups that can be used for the purpose of the present invention include a methyl group, an ethyl group, a propyl group, an isopropyl group, a butyl group, an isobutyl group, a sec-butyl group, a tert-butyl group, a pentyl group, an isopentyl group, a 1-methylbutyl group, a 2-methylbutyl group, a tert-pentyl group, a hexyl group, a cyclopropyl group, a cyclobutyl group, a cyclopentyl group, a cyclohexyl group, a cycloheptyl group and a cyclooctyl group.

The aralkyl group has preferably 7 carbon atoms to 19 carbon atoms, more preferably 7 carbon atoms to 15 carbon atoms. Examples of aralkyl groups that can be used for the purpose of the present invention include a benzyl group, a phenethyl group, a phenylpropyl group and a naphthylmethyl group.

The aryl group has preferably 6 carbon atoms to 18 carbon atoms, more preferably 6 carbon atoms to 14 carbon atoms. Examples of aryl groups that can be used for the purpose of the present invention include a phenyl group, a naphthyl group, an anthryl group and an azulenyl group.

Examples of halogen atoms that can be used for the purpose of the present invention include a chlorine atom, a bromine atom, a fluorine atom and an iodine atom.

The aromatic ring formed by adjacently located two R⁶ together with two adjacent carbon atoms preferably has 6 carbon atoms to 14 carbon atoms. Examples of aromatic rings that can be used for the purpose of the present invention include a benzene ring and a naphthalene ring.

Examples of heterocyclic ring in the heterocyclic group include a 5-membered or 6-membered monocyclic aromatic or alicyclic heterocyclic ring containing at least an atom selected from a nitrogen atom, an oxygen atom and a sulfur atom and a bicyclic or tricyclic condensed aromatic or alicyclic heterocyclic ring formed by condensing a 3- to 8-membered rings and containing at least an atom selected from a nitrogen atom, an oxygen atom and a sulfur atom.

Specific examples of heterocyclic rings in the heterocyclic group include a pyridine ring, a pyrazine ring, a pyrimidine ring, a pyridazine ring, a quinoline ring, an isoquinoline ring, a phthalazine ring, a quinazoline ring, a quinoxaline ring, a naphthyridine ring, a cinnoline ring, a pyrrol ring, a pyrazol ring, an imidazole ring, a triazole ring, a tetrazole ring, a thiophene ring, a furan ring, a thiazole ring, an oxazole ring, an indole ring, an isoindole ring, an indazole ring, a benzimidazole ring, a benzotriazole ring, a benzothiazole ring, a benzoxazole ring, a purine ring, a carbazole ring, a pyrrolidine ring, a piperidine ring, a piperazine ring, a morpholine ring, a thiomorpholine ring, a homopiperidine ring, a homopiperazine ring, tetrahydropyridine ring, a tetrahydroquinoline ring, a tetrahydroisoquinoline ring, a tetrahydrofuran ring, a tetrahydropyran ring, a dihyrobenzofuran ring and a tetrahydrocarbazole ring.

The substituent of the aralkyl group, the aryl group, the alkoxy group, the heterocyclic group or the aromatic ring formed by two adjacently located R⁶s together with respective two adjacent carbon atoms may be a group of 1 substituent to 5 substituents that may be the same or different, which may be selected from a group including a hydroxyl group, a carboxyl group, a halogen atom, an alkyl group, an alkoxy group, a nitro group and a substituted or unsubstituted amino group. Examples of halogen atoms, alkyl groups and alkoxy groups are the same as those listed above.

The substituent of the alkyl group may be a group of 1 substituent to 3 substituents that may be the same or different, which may be selected from a group including a hydroxyl group, a carboxyl group, a halogen atom and an alkoxy group. Examples of halogen atoms and alkoxy groups are the same as those listed above.

The substituent of the amino group includes one or two alkyl groups which may be the same or different. Examples of the alkyl groups are the same as those listed above.

A squarylium metal chelate compound expressed by the general formula (I) and (II) can be manufactured by applying a method described in WO02/50190 Publication. Now, a synthesis method for preparing a squarylium compound expressed by the general formula (I) will be described below in detail.

A compound expressed by the general formula (VII) below can be manufactured by applying a method described in Japanese Patent Application Laid-Open (JP-A) 2005-53875.

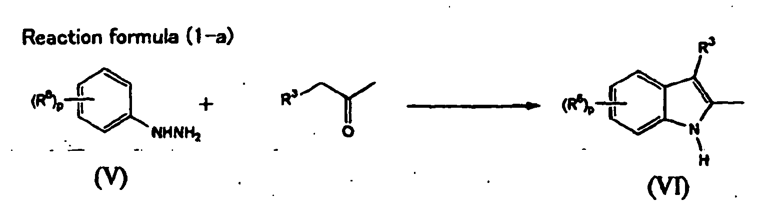

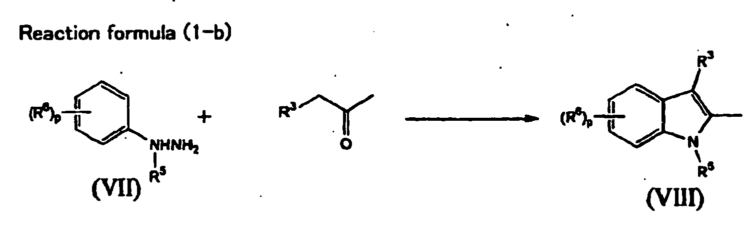

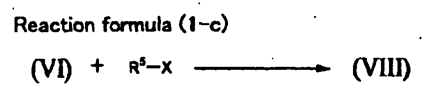

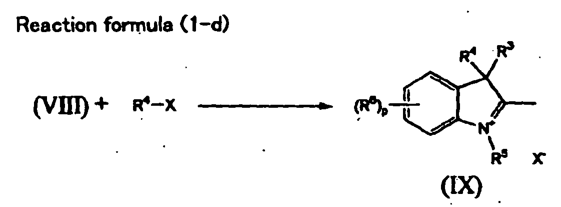

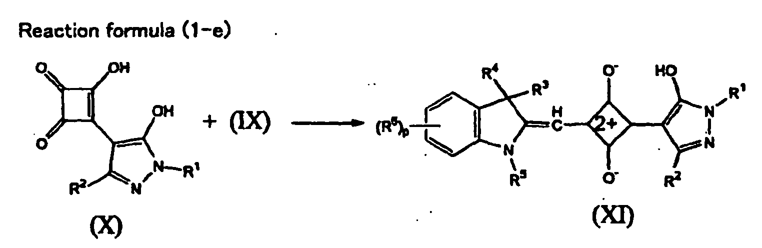

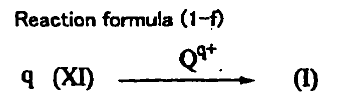

In the formulae above, R¹, R², R³, R⁴, R⁵, R⁶, Q and q are the same as those in the preceding formulas and X represents halogen atoms, an alkylsulfonyl group that may have a substituent or an arylsulfonyl group that may have a substituent. The alkyl group and the aryl group are the same as those in the preceding formulas.

### Reaction formula (1 - a):

The compound (VI) is obtained by reacting the compound (V) and benzylacetone by mols 0.7 times to 1.5 times as many as those of the compound (V) in a solvent in the presence of acid at 50°C to 120°C for from 5 minutes to 15 hours. The acid is selected from a group including hydrochloric acid, sulfuric acid, acetic acid, p-toluenesulfonic acid and benzenesulfonic acid. The amount of the acid that is put to use is preferably such that the mols of the acid are 0.7 times to 1.5 times as many as those of the compound (V). The solvent is selected from a group including alcohol type solvents such as ethanol, propanol, isopropanol, butanol and isobutanol and aromatic type solvents such as benzene, toluene and xylene.

### Reaction formula (1 - b):

The compound (VIII) is obtained by causing the compound (VII) and benzylacetone to react with each other in a solvent in the presence of acid at 50°C to 120°C for from 5 minutes to 15 hours. The acid is selected from a group including hydrochloric acid, sulfuric acid, acetic acid, p-toluenesulfonic acid and benzenesulfonic acid. The amount of acid that is put to use is preferably such that the mols of the acid are 0.7 times to 1.5 times as many as those of the compound (VII). The solvent is selected from a group including alcohol type solvents such as ethanol, propanol, isopropanol, butanol and isobutanol and aromatic type solvents such as benzene, toluene and xylene.

### Reaction formula (1 - c):

The compound (VIII) is obtained by causing the compound (VI) and R⁵-X by mols 0.7 times to 10 times as many as those of the compound (VI) to react with each other in a solvent in the presence of base at 0°C to 80°C for from 5 minutes to 15 hours. The base is selected from a group including sodium hydroxide, potassium hydroxide, sodium methoxide, potassium t-butoxide and sodium hydride. The amount of base that is put to use is preferably such that the mols of the base are 0.7 times to 10 times as many as those of the compound (VI). The solvent is selected from a group including halogen type solvents such as chloroform and dichloromethane, aromatic type solvents such as benzene, toluene and xylene, ether type solvents such as tetrahydrofuran and methyl-tert-butyl ether, dimethylsulfoxide and dimethylformamide.

### Reaction formula (1 - d):

The compound (IX) is obtained by causing the compound (VIII) and R⁴-X by mols 0.7 times to 10 times as many as those of the compound (VIII) to react with each other in a solvent at 50°C to 150°C for from 5 minutes to 30 hours. The solvent is selected from a group including alcohol type solvents such as ethanol, propanol, isopropanol, butanol and isobutanol, aromatic type solvents such as benzene, toluene and xylene, halogen type solvents such as chloroform and dichloromethane, ether type solvents such as tetrahydrofuran and methyl-tert-butyl ether.

### Reaction formula (1- e):

The compound (XI) is obtained by causing the compound (X) and the compound (IX) by mols 0.5 times to 2 times as many as those of the compound (X), that is in the presence of base by mols 0.5 times to 2 times as many as those of the compound (X), to react with each other in a solvent at 50°C to 120°C for from 5 minutes to 15 hours. The solvent is selected from a group including alcohol type solvents such as ethanol, propanol, isopropyl alcohol, butanol and octanol, mixture solvents of an alcohol type solvent as listed above and an aromatic type solvent such as benzene, toluene or xylene (preferably the alcohol type solvent 50 volume/volume% or more). The base is selected from a group including organic bases such as quinoline, triethylamine and pyridine and inorganic bases such as potassium carbonate, potassium bicarbonate and sodium bicarbonate.

### Reaction formula (1 - f):

The compound (I) is obtained by causing the compound (XI) and a material that provides Q^{q+}, if necessary in the presence of acetic acid by mols 0.5 times to 2 times as many as those of the compound (XI), to react with each other in a solvent at room temperature to 120°C for from 5 minutes to 15 hours. The material that provides Q^{q+} is preferably used in such an amount that ratio of the number of mols of the compound (XI) : the number of mols x q of the material that gives rise to □Q^{q+} is 1 : 0.5 to 2.

Examples of materials that give rise to Q^{q+} include aluminum tris(acetylacetonate), aluminum-tris(ethylacetonate), aluminum isoproxide, aluminum sec-butoxide, aluminum ethoxide, aluminum chloride, copper chloride, copper acetate and nickel acetate.

Examples of solvents that can be used for this reaction include halogen type solvents such as chloroform and dichloromethane, aromatic type solvents such as benzene, toluene and xylene, ether type solvents such as tetrahydrofuran and methyl-tert-butyl-ether and ester type solvents such as ethyl acetate.

Specific examples of the compound (XI) that can be obtained by way of the above-described reactions are listed in Tables 1 through 3 below. Note that the compound numbers in the tables are used as compound numbers of ligand in Examples, which will be described hereinafter. The compound (I) is suffixed by a metal atom. For example, a squarylium compound forming an aluminum complex that is attributed to the compound 1 is expressed as the Compound 1-Al (No. 1-Al). In the tables, Ph denotes a phenyl group.

**Table 1**

| Specific examples of compound (XI) | |
|---|---|
| No. | Squarylium Compound |
| 1 | 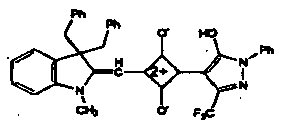 |
| 2 | 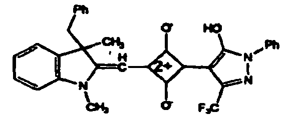 |
| 3 | 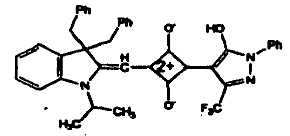 |
| 4 | 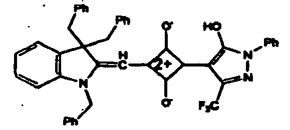 |
| 5 | 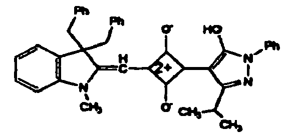 |
| 6 | 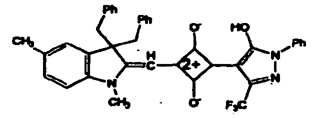 |
| 7 | 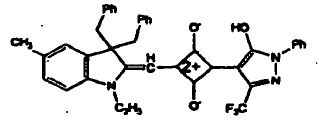 |

**Table 2**

| Specific examples of compound (XI) | |
|---|---|
| No. | Squrylium compound |
| 8 | 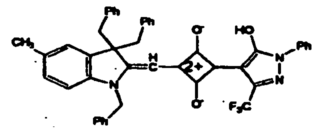 |
| 9 | 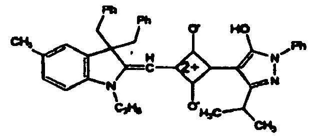 |
| 10 | 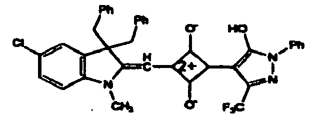 |
| 11 | 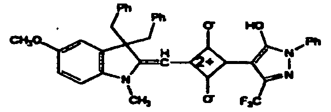 |
| 12 | 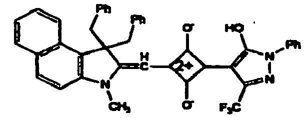 |
| 13 | 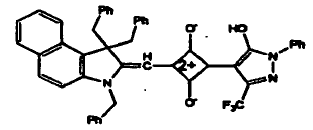 |
| 14 | 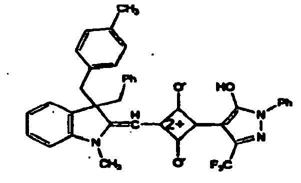 |

**Table 3**

| Specific examples of compound (XI) | |
|---|---|
| No. | Squrylium compound |
| 15 | 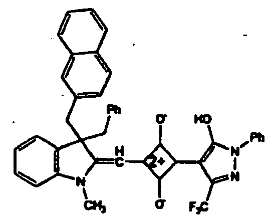 |
| 16 | 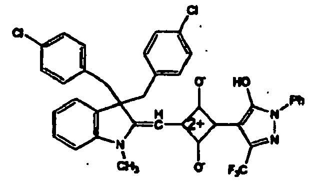 |
| 17 | 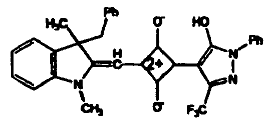 |
| 18 | 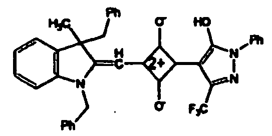 |
| 19 | 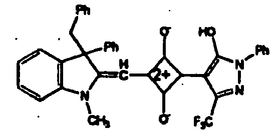 |
| 20 | 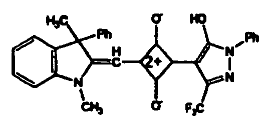 |

It is preferable that the recording layer that is made of a squarylium metal chelate compound expressed by the general formula (I) or the recording layer that is formed by adding a squarylium metal chelate compound expressed by the general formula (II) to a compound expressed by the general formula (I) of an optical recording medium, which will be described in greater detail hereinafter, further contains a formazan metal chelate compound forming a metal complex of a formazan compound expressed by the general formula (III) or the general formula (IV) in order to improve the storage stability of the recording medium.

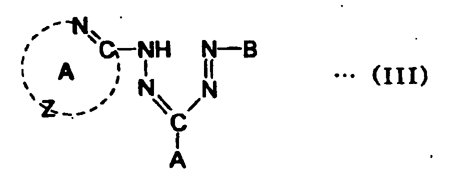

In the general formula (III), ring A represents a substituted or unsubstituted 5-membered or 6-membered ring containing a nitrogen atom; Z represents an atom group forming the ring A wherein the nitrogen-containing heterocyclic ring is optionally condensed with some other ring; A represents a substituted or unsubstituted alkyl group, a substituted or unsubstituted aryl group, a substituted or unsubstituted alkylcarbonyl group, a substituted or unsubstituted arylcarbonyl group, a substituted or unsubstituted alkenyl group, a substituted or unsubstituted heterocyclic ring residue, or a substituted or unsubstituted alkyloxycarbonyl group; and B represents a substituted or unsubstituted alkyl group, a substituted or unsubstituted alkenyl group, or a substituted or unsubstituted aryl group.

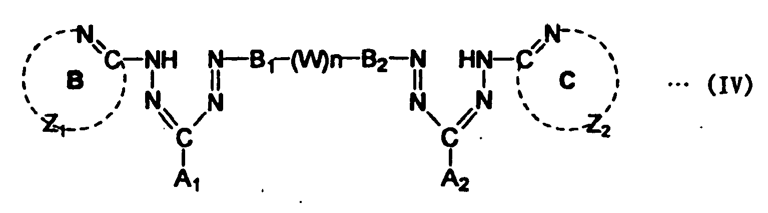

In the general formula (IV), ring B and ring C are the same or different to each other and each of ring B and ring C represents a substituted or unsubstituted 5-membered or 6-membered ring containing a nitrogen atom; Z₁ and Z₂ respectively represent atom groups forming the ring B and the ring C, and each of the nitrogen containing heterocyclic rings is optionally condensed with some other ring; each of A₁ and A₂ represents a substituted or unsubstituted alkyl group, a substituted or unsubstituted aryl group, a substituted or unsubstituted alkylcarbonyl group, a substituted or unsubstituted arylcarbonyl group, a substituted or unsubstituted alkenyl group, a substituted or unsubstituted heterocyclic ring residue, or a substituted or unsubstituted alkyloxycarbonyl group; and each of B₁ and B₂ represents a substituted or unsubstituted alkylene group, a substituted or unsubstituted alkenylene group, or a substituted or unsubstituted arylene group; W represents -CH₂- or -SO₂-; and n represents an integer of 0 or 1.

Each of the ring A, ring B and ring C in the above formulas represents a substituted or unsubstituted 5-membered or 6-membered ring that contains a nitrogen atom and Z, **Z₁** and Z₂ respectively represent atom groups forming the ring A, the ring B and the ring C. Any of the atom groups may contain one or more hetero atoms beside carbon atoms. For the purpose of the present invention, hetero atoms include nitrogen atom (-N-), sulfur atom (-S-), oxygen atom (-O-) and selenium atom (-Se-).

Some other ring D may be bonded to any of the ring A, the ring B and the ring C. Such a ring D may be a carbon ring or a heterocyclic ring. If the ring D is a carbon ring, the number of the ring-constituting carbon atoms is preferably 6 to 20, more preferably 6 to 10. Specific examples of such a ring include benzene ring, naphthalene ring and cyclohexane ring. In the case of a heterocyclic ring, the number of the ring-constituting atoms is preferably 5 to 20, more preferably 5 to 14. Specific examples of such a ring include pyrrolidine, thiazole, imidazole, oxazole, pyrazole, pyridine, pyridazine, pyrimidine, pyrazine, quinoline, indoline and carbazole.

Specific examples of the ring A, the ring B and the ring C include thiazole, imidazole, thiadiazole, oxazole, triazole, pyrazole, oxadiazole, pyridine, pyridazine, pyrimidine, pyrazine and triazine.

Examples of the substituent that can be bonded to the ring A, the ring B and the ring C independently include halogen atoms, nitro group, cyano group, hydroxyl group, carboxyl group, amino group, carbamoyl group, alkyl groups that may have a substituent, aryl groups that may have a substituent, heterocyclic groups that may have a substituent, alkoxy groups that may have a substituent, aryloxy groups that may have a substituent, alkylthio groups that may have a substituent, arylthio groups that may have a substituent, alkylamino groups that may have a substituent, arylamino groups that may have a substituent, alkoxycarbonyl groups that may have a substituent, aryloxycarbonyl groups that may have a substituent, alkylcarboxamide groups that may have a substituent, arylcarboxamide groups that may have a substituent, alkylcarbamoyl groups that may have a substituent, arylcarbamoyl groups that may have a substituent, alkenyl groups that may have a substituent and alkylsulfamoyl groups that may have a substituent.

Each of A, A₁ and A₂ in the above general formulas (III) and (IV) represents an alkyl group that may have a substituent, an aryl group that may have a substituent, an alkylcarbonyl group that may have a substituent, an arylcarbonyl group that may have a substituent, an alkenyl group that may have a substituent, a heterocyclic group that may have a substituent or an alkoxycarbonyl group that may have a substituent. If it is an alkyl group or an alkenyl group, it may have a chain and/or a ring. If it is an alkyl group, the number of carbon atoms is preferably 1 to 15, more preferably 1 to 8. If it is an alkenyl group, the number of carbon atoms is preferably 2 to 8, more preferably 2 to 6.

In the above general formula (III), B represents an alkyl group that may have a substituent, an alkenyl group that may have a substituent or an aryl group that may have a substituent. If it is an alkyl group or an alkenyl group, it may have a chain and/or a ring. If it is an alkyl group, the number of carbon atoms is preferably 1 to 15, more preferably 1 to 8. If it is an alkenyl group, the number of carbon atoms is preferably 2 to 8, more preferably 2 to 6. If it is an aryl group, the number of carbon atoms is preferably 6 to 18, more preferably 6 to 14.

Each of B₁ and B₂ in the above general formula (IV) represents an alkylene group that may have a substituent, an alkenylene group that may have a substituent or an arylene group that may have a substituent. The alkylene group or the alkenylene group may have a chain and/or a ring. If it is an alkylene group, the number of carbon atoms is preferably 1 to 15, more preferably 1 to 8. If it is an alkenylene group, the number of carbon atoms is preferably 2 to 8, more preferably 2 to 6. If it is an arylene group, the number of carbon atoms is preferably 6 to 18, more preferably 6 to 14.

If the above general formulas (III) and (IV) contain an alkyl group, the number of carbon atoms of the alkyl group is preferably 1 to 15. Specific examples of alkyl groups having such a number of carbon atoms include straight chain alkyl groups such as a methyl group, an ethyl group, an n-propyl group, an n-butyl group, an n-pentyl group, an n-hexyl group, an n-heptyl group, an n-octyl group, an n-nonyl group and an n-decyl group, branched chain alkyl groups such as an isobutyl group, an isoamyl group, a 2-methylbutyl group, a 2-methylpentyl group, a 3-methylpentyl group, a 4-methylpentyl group, a 2-ethylbutyl group, a 2-methylhexyl group, a 3-methylhexyl group, a 4-methylhexyl group, a 5-methylhexyl group, a 2-ethylpentyl group, a 3-ethylpentyl group, a 2-methylheptyl group, a 3-methylheptyl group, a 4-methylheptyl group, a 5-methyheptyl group, a 2-ethylhexyl group, a 3-ethylhexyl group, a isopropyl group, a sec-butyl group, a 1-ethylpropyl group, a 1-methylbutyl group, a 1,2-dimethylpropyl group, a 1-methylheptyl group, a 1-ethylbutyl group, a 1,3-dimethylbutyl group, a 1,2-dimethylbutyl group, a 1-ethyl-2-methylpropyl group, a 1-methylhexyl group, a 1-ethylheptyl group, 1-propylbutyl group, a 1-isopropyl-2-methylpropyl group, a 1-ethyl-2-methylbutyl group, a 1-propyl-2-methylpropyl group, a 1-methylheptyl group, a 1-ethylhexyl group, a 1-propylpentyl group, a 1-isopropylpentyl group, a 1-isopropyl-2-methylbutyl group, a 1-isopropyl-3-methylbutyl group, a 1-methyoctyl group, a 1-ethylheptyl group, a 1-propylhexyl group, a 1-isobutyl-3-methylbutyl group, a neopentyl group, a tert-butyl group, a tert-hexyl group, a tert-amyl group and as tert-branched alkyl group such as an octyl group and cycloalkyl groups such as a cyclohexyl group, a 4-methylcyclohexyl group, a 4-ethylcyclohexyl group, a 4-tert-butylcyclohexyl group, a 4-(2-ethylhexyl)cyclohexyl group, a bornyl group, a isobornyl group and an adamantyl group. A group having 1 to 8 carbon atoms is preferably employed for the purpose of the present invention.

For the purpose of the present invention, the alkyl groups that can be used for the general formulas (III) and (IV) may be substituted by a hydroxyl group, a halogen atom, a nitro group, a carboxyl group, a cyano group or the like. Alternatively, it may be substituted by an aryl group or a heterocyclic group that may have a specific substituent (and hence may be substituted, for example, by a halogen atom or a nitro group). Still alternatively, it may be substituted by some other hydrocarbon group such as an alkyl group, which may be any of the above-listed ones, by way of a hetero atom such as oxygen, sulfur or nitrogen.

Examples of alkyl groups substituted by some other hydrocarbon group by way of oxygen include alkyl groups substituted by an alkoxy group or an aryloxy group such as a methoxymethyl group, a methoxyethyl group, an ethoxymethyl group, an ethoxyethyl group, a butoxyethyl group an ethoxyethoxyethyl group, a phenoxyethyl group, a methoxypropyl group and an ethoxypropyl group. Such alkoxy groups and aryloxy groups may have a substituent.

Examples of alkyl groups substituted by some other hydrocarbon group by way of sulfur include alkyl groups substituted by an alkylthio group or an arylthio group such as a methylthioethyl group, an ethylthioethyl group and a phenylthioethyl group. Such alkylthio groups and arylthio groups may have a substituent.

Examples of alkyl groups substituted by some other hydrocarbon group by way of nitrogen include alkyl groups substituted by any of an alkylamino group or an arylamino group such as a dimethylaminoethyl group, a diethylaminoethyl group, a diethylaminopropyl group and a phenylaminomethyl group. Such alkylamino groups and arylamino groups may have a substituent.

If the above general formulas (III) and (IV) contain an alkenyl group, the number of carbon atoms of the alkenyl group is preferably 2 to 6. Specific examples of alkenyl groups having such a number of carbon atoms include a vinyl group, an allyl group, a 1-propenyl group, a methacryl group, a crotyl group, a 1-butenyl group, a 3-butenyl group, a 2-pentenyl group, a 4-pentenyl group, a 2-hexenyl group, a 5-hexenyl group, a 2-heptenyl group and a 2-octenyl group. Examples of substituents for the alkenyl group include those listed above for the alkyl group.

Specific examples of aryl groups include a phenyl group, a naphthyl group, an anthranyl group, a fluorenyl group, phenalenyl group, phenanthranyl group, triphenylenyl group, and a pyrenyl group.

Examples of alkylene groups and those of alkenylene groups include groups obtained by removing a hydrogen atom respectively from the alkyl groups and the alkenyl groups that are listed above.

Examples of arylene groups include groups obtained by removing a hydrogen atom from the above-listed aryl groups.

For the purpose of the present invention, the aryl groups and the arylene groups that can be used for the general formulas (III) and (IV) may be substituted by an alkyl group, an alkenyl group, a hydroxyl group, a halogen atom, a nitro group, a carboxyl group, a cyano group, a trifluoromethyl group, an aryl group that may have a specific substituent (and hence may be substituted, for example, by a halogen atom or a nitro group) or a heterocyclic group that may have a specific substituent (and hence may be substituted, for example, by a halogen atom or a nitro group). Examples of alkyl groups, those of alkenyl groups and those of aryl groups include those listed above. Examples of halogen atoms are the same as those that will be described hereinafter.

Specific examples of heterocyclic groups include a furyl group, a thienyl group, a pyrrolyl group, a benzofuranyl group, an isobenzofuranyl group, a benzothienyl group, an indolinyl group, an isoindolinyl group, a carbazolyl group, a pyridyl group, piperidyl group, a quinolyl group, an isoquinolyl group, an oxazolyl group, an isooxazolyl group, a thiazolyl group, an isothiazolyl group, an imidazolyl group, a pyrazolyl group, a benzoimidazolyl group, a pyrazyl group, a pyrimidinyl group, a pyridazinyl group and a quinoxalinyl group.

The above-listed heterocyclic groups may be substituted by a hydroxyl group, an alkyl group, a halogen atom, a nitro group, a carboxyl group, a cyano group, an aryl group that may have a specific substituent (and hence may be substituted by a halogen atom or a nitro group) or a heterocyclic group that may have a specific substituent (and hence may be substituted, for example, by a halogen atom or a nitro group). Still alternatively, it may be substituted by some other hydrocarbon group such as alkyl group, which may be any of the above-listed one, by way of a hetero atom such as oxygen, sulfur or nitrogen. Examples of alkyl groups, those of alkenyl groups and those of aryl groups include those listed above. Examples of halogen atoms are the same as those that will be described hereinafter.

As for the substituent that can be bonded to the ring A, the ring B and/or the ring C, specific examples of the halogen atom include fluorine, chlorine, bromine and iodine.

It is sufficient for the alkoxy group that may have a substituent that an alkyl group that may directly have a substituent at the oxygen atom thereof is bonded to it. Specific examples of alkyl groups and those of substituents include those listed above.

It is sufficient for the aryloxy group that may have a substituent that an aryl group that may directly have a substituent at the oxygen atom thereof is bonded to it. Specific examples of aryl groups and those of substituents include those listed above.

It is sufficient for the alkylthio group that may have a substituent that an alkyl group that may directly have a substituent at the sulfur atom thereof is bonded to it. Specific examples of alkyl groups and those of substituents include those listed above.

It is sufficient for the arylthio group that may have a substituent that an aryl group that may directly have a substituent at the sulfur atom thereof is bonded to it. Specific examples of aryl groups and those of substituents include those listed above.

It is sufficient for the alkylamino group that may have a substituent that an alkyl group that may directly have a substituent at the nitrogen atom thereof is bonded to it. Specific examples of alkyl groups and those of substituents include those listed above. Alternatively, the alkyl groups may be bonded to each other and contain an oxygen atom and a nitrogen atom to form a ring such as a piperidino group, a morpholino group, a pyrrolidinyl group, a piperazinyl group, an indolinyl group, an isoindolinyl group or the like.

It is sufficient for the arylamino group that may have a substituent that an aryl group that may directly have a substituent at the nitrogen atom thereof is bonded to it. Specific examples of aryl groups and those of substituents include those listed above.

It is sufficient for the alkylcarbonyl group that may have a substituent that an alkyl group that may directly have a substituent at a carbon atom of the carbonyl group thereof is bonded to it. Specific examples of alkyl groups and those of substituents include those listed above.

It is sufficient for the arylcarbonyl group that may have a substituent that an aryl group that may directly have a substituent at a carbon tom of the carbonyl group thereof is bonded to it. Specific examples of aryl groups and those of substituents include those listed above.

It is sufficient for the alkoxycarbonyl group that may have a substituent that an alkyl group that may directly have a substituent at the oxygen atom thereof is bonded to it. Specific examples of alkyl groups and those of substituents include those listed above.

It is sufficient for the aryloxycarbonyl group that may have a substituent that an aryl group that may directly have a substituent at the oxygen atom thereof is bonded to it. Specific examples of aryl groups and those of substituents include those listed above.

It is sufficient for the alkylcarboxamide group that may have a substituent that an alkyl group that may directly have a substituent at a carbon atom of the carboxamide thereof is bonded to it. Specific examples of alkyl groups and those of substituents include those listed above.

It is sufficient for the arylcarboxamide groups that may have a substituent that an aryl group that may directly have a substituent at a carbon atom of the carboxamide thereof is bonded to it. Specific examples of aryl groups and those of substituents include those listed above.

It is sufficient for the alkylcarbamoyl group that may have a substituent that an alkyl group that may directly have a substituent at the nitrogen atom of the carbamoyl group thereof is bonded to it. Specific examples of alkyl groups and those of substituents include those listed above. Alternatively the alkyl groups may be bonded to each other and contain an oxygen atom and a nitrogen atom to form a ring such as a piperidino group, a morpholino group, a pyrrolidinyl group, a piperazinyl group, an indolinyl group, an isoindolinyl group or the like.

It is sufficient for the arylcarbamoyl group that may have a substituent that an aryl group that may directly have a substituent at the nitrogen atom of the carbamoyl group thereof is bonded to it. Specific examples of aryl groups and those of substituents include those listed above.

It is sufficient for the alkylsulfamoyl group that may have a substituent that an alkyl group that may directly have a substituent at the nitrogen atom of the sulfamoyl group thereof is bonded to it. Specific examples of alkyl groups and those of substituents include those listed above.

It is sufficient for the metal component of the formazan metal chelate derived from the compound expressed by the general formula (III) or (VI) that it is a metal or a metal compound that can form a chelate at the formazan. Examples of such metal components include titanium, vanadium, chromium, manganese, iron, cobalt, nickel, copper, zinc, zirconium, niobium, molybdenum, technetium, ruthenium, rhodium, palladium, oxides of the above metals and halides of the above metals. Preferable metals for the purpose of the present invention include vanadium, manganese, iron, cobalt, nickel, copper, zinc and palladium. An optical recording medium formed by using a formazan metal chelate compound according to the present invention shows excellent optical characteristics. As halides of the above metals, chlorides of the metals are preferably used for the purpose of the present invention.

Now, an optical recording medium according to the present invention will be described below.

An optical recording medium according to the present invention contains a recording layer containing a squarylium metal chelate compound (organic dye) that is expressed by the above general formula (I) and provides good recording signal characteristics at high-speed recording and an excellent resistance against light. An optical recording medium according to the present invention can suitably find applications in one-side single-layer recording mediums and one-side double-layer recording mediums such as DVDs.

A one-side single-layer recording medium has a recording layer that is formed on a substrate and contains an organic dye. If necessary, it has a reflective layer and a protective layer formed on the other side.

A one-side double-layer recording medium has a first information layer and a second information layer with an intermediate layer interposed between them. If necessary, it has one or more additional layers.

The first embodiment of the first information layer of a one-side double-layer optical recording medium according to the present invention includes a first substrate and a read-only first recording layer that has information pits and is made of a reflective film formed on the first substrate. If necessary, the embodiment has one or more additional layers. The first information layer of a one-side double-layer optical recording medium according to the present invention includes a first substrate and a first recording layer at least containing an organic dye and a first reflective layer (translucent) formed on the first substrate in the mentioned order. If necessary, the embodiment has one or more additional layers such as a protection layer, an undercoat layer and/or a hard coat layer. More specifically, the first information layer is the same as the information layer of a one-side single-layer optical recording medium except that the reflective layer is translucent.

The second information layer of a one-side double-layer optical recording medium according to the present invention includes a second substrate and at least a reflective layer, the second recording layer containing an organic dye and a protection layer formed on the second substrate in the mentioned order. If necessary, the embodiment has one or more additional layers such as a protection layer, an undercoat layer and/or a hard coat layer.

With an optical recording medium according to the present invention, light having a recording wavelength of 580 nm to 720 nm is irradiated from the side of the surface of the first substrate to at least record signal information on the first recording layer and the second recording layer or reproduce signal information from the first recording layer and the second recording layer.

When recording marks are to be formed on the second recording layer of a double-layer type optical recording medium having the first recording layer and the second recording layer by means of the recording/reproduction light attenuated by the first recording layer and the reflective layer (or the second recording layer), the attenuation of light due to the absorption of light by the first recording layer is remarkable when compared with a double-layer ROM (such as a DVD-ROM). Then, as a result, it is difficult to provide a satisfactory level of recording power at the second recording layer. Additionally, a focus offset is apt to arise at the second recording layer due to optical aberration to broaden recording marks, which by turn can give rise to increased cross talks with adjacent tracks. Still additionally, the profile of the groove or grooves of the second substrate can be a factor of increasing cross talks. More specifically, when the polarities of the undulations of the second recording layer are made to match each other as viewed from the light receiving surface, this arrangement does not provide any effect of preventing recording marks from being broadened by the groove or grooves because recording marks are formed on the inter-groove section or sections (on the ridge or ridges).

When forming recording marks on the inter-groove section or sections, it is necessary to make the groove or grooves of the second recording layer less deep than the groove or grooves of the first recording layer in order to realize a reflectance similar to that of the first recording layer because the main reflective surface of the second recording layer is formed on the interface of the recording layer and the reflective surface (or the second reflective layer) so that cross talks are apt to increase. For these reasons, jitters can increase due to broadened recording marks particularly when signals are being recorded at high-speed (15 m/s or higher), while such a high-speed recording requires high power.

However, the above-identified problems can be solved by forming a recording layer that contains a squarylium compound forming a metal complex (squarylium metal chelate compound) according to the present invention that is expressed by the general formula (I).

For example, to realize a desired recording sensitivity and suppress broadening of recording marks at the second recording layer in order to achieve good signal characteristics according to the present invention, it is preferable that the second recording layer contains at least a squarylium metal chelate compound selected from squarylium metal chelate compounds having a specific structure according to the present invention and a protection layer (deformation prevention layer for preventing thermal deformations) that is contiguous to the second recording layer is formed. Additionally, it is preferable that the guide groove of the second substrate is made to show an appropriate depth in order to get a high reflectance from the second recording layer.

Now, the configuration of a one-side double-layer optical recording medium according to the present invention will be described below by referring to the related drawings.

FIG. 2 is a schematic cross sectional view of the first embodiment of optical recording medium according to the present invention, showing the layer arrangement thereof.

In the instance of a layer arrangement illustrated in FIG. 2, the first recording layer 203 is a layer dedicated to reproduction and the second recording layer 207 is a layer dedicated to recording and reproduction. In FIG. 2, 201 denotes the first substrate and 202 denotes the second substrate, while 203 denotes the first read only recording layer that is made of reflective film and 205 and 206 respectively denote the intermediate layer and the protection layer. In FIG. 2, 207 denotes the second recording layer that contains an organic dye and 208 denotes the reflective layer. Light is irradiated from the side of the first substrate for recording/reproduction.

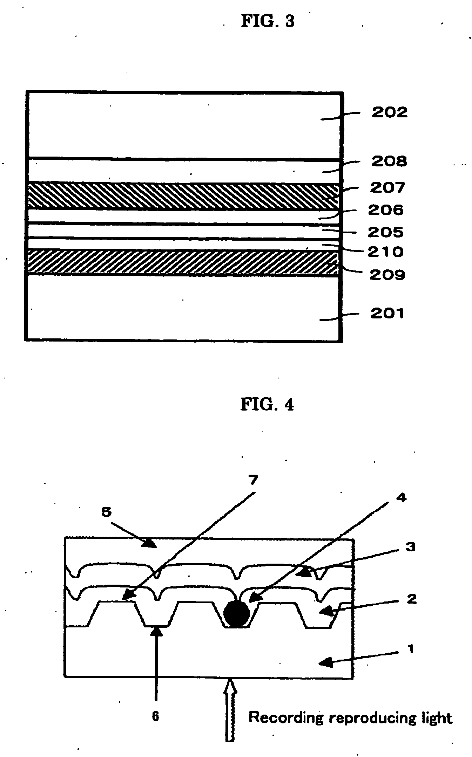

FIG. 3 is a schematic cross sectional view of the second embodiment of an optical recording medium according to the present invention, showing the layer arrangement thereof.

In the instance of layer arrangement illustrated in FIG. 3, both the first recording layer 203 and the second recording layer 207 are used for both recording and reproduction unlike their counterparts of FIG. 2.

In FIG. 3, 201 and 209 respectively denote the first substrate and the first recording layer that contains an organic dye and 210 and 202 respectively denote the first reflective layer and the second substrate, while 205 and 206 respectively denote the intermediate layer and the protection layer and 207 and 208 respectively denote the second recording layer that contains an organic dye and the second reflective layer. Light is irradiated from the side of the first substrate for recording/reproduction.

According to the present invention, it is also possible to form a single-layered recording layer medium that does not have the first recording layer unlike the recording mediums illustrated in FIGS. 2 and 3.

Like a DVD+R and a CD-R, an optical recording medium according to the present invention provides a high reflectance due to the multi-interference effect of the two interfaces of the recording layers that contain an organic dye (dye recording layers). It is preferable that the recording layers show optical characteristics including a large refractive index n and a relatively small extinction coefficient k. Particularly, it is preferable that n > 2 and 0.03 < k < 0.2. Such optical characteristics can be obtained by utilizing the characteristics of an end part of the long wavelength side of the light absorption band of the dye-containing recording layers.

Now, the layer arrangement of an optical recording medium according to the present invention will be described in greater detail below.

### [Recording layers]

Of the recording layers, the first recording layer of the first information layer may include a reflective film that is dedicated to reproduction or contains an organic dye. The reflective film is made of a metal or an alloy that is similar to the material of the reflective layer that will be described hereinafter and has information pits formed therein.

The second recording layer of the second information layer contains an organic dye expressed by the structural formula (I).

For the purpose of the present invention, the thermal decomposition temperature of the organic dye (squarylium metal chelate compound expressed by the general formula (I)) in the recording layer or layers is preferably between 200°C and 350°C. The recording power can fall when the thermal decomposition temperature exceeds 350°C, whereas the thermal stability tends to fall and the recording marks become apt to be broadened when the thermal decomposition temperature is below 200°C. The thermal decomposition temperature of the organic dye can be observed with ease by means of a general purpose thermobalance.

While the organic dye of the first recording layer preferably contains an organic dye same as that of the second recording layer (a squarylium metal chelate compound expressed by the general formula (I)), it is possible to appropriately select some other material according to the application.

Examples of such materials include cyanine dyes, tetraazaporphyrazine dyes, phthalocyanine dyes, pyrylium dyes, thiopyrylium dyes, azulenium dyes, squarylium dyes, squarylium metal chelate compounds, formazan chelate dyes, metal complex dyes of metals such as Ni and Cr, naphthoquinone/anthraquinone dyes, indophenol dyes, indoaniline dyes, triphenylmethane dyes, triarylmethane dyes, aluminum/diimmonium dyes, nitroso compounds. Any of these compounds may be used alone or two or more of them may be combined for use. If necessary, any of the above-listed materials may be added to the second recording layer.

Of the above-listed materials, at least a dye selected from tetraazaporphyrazine dyes, cyanine dyes, azo dyes, squarylium dyes, squarylium metal chelate compounds and formazan chelate dyes is preferably used for the purpose of the present invention from the viewpoint that, with such a material, the maximum absorption wavelength or the absorption peak wavelength of the light absorption spectrum of the organic-dye-containing recording layer is found within the range of 580 nm to 630 nm to make it easy to obtain desired optical characteristics with a laser beam wavelength (of about 650 nm), from a film by applying solvent and adjusting the optical characteristics.

The film thickness of the first recording layer is preferably from 10 nm to 500 nm (100Å to 5,000Å), more preferably between 40 nm to 80 nm (400Å and 800Å). The recording sensitivity can fall when the film thickness of the first recording layer is less than 10 nm, whereas the reflectance can fall when the film thickness exceeds 500 nm.

The film thickness of the second recording layer of a one-side double-layer recording medium according to the present invention is preferably between 40 nm to 100 nm (400Å to 1,000Å), more preferably between 50 nm to 80 nm. It may not be possible to obtain good jitter characteristics when the film thickness of the second recording layer is less than 40 nm, whereas both cross talks and jitters can increase when the film thickness exceeds 100 nm. The ratio of the film thickness of the second recording layer in the guide groove section to the film thickness of the second recording layer in the inter-guide-groove section (recording guide groove section / inter-recording-guide-groove section) is preferably within the range of 80% to 100%.

The first recording layer and the second recording layer can be formed by any ordinary means such as evaporation, sputtering, CVD (chemical vapor deposition) or application of solution. They can be manufactured by a method of manufacturing an optical recording medium according to the invention as will be described hereinafter.

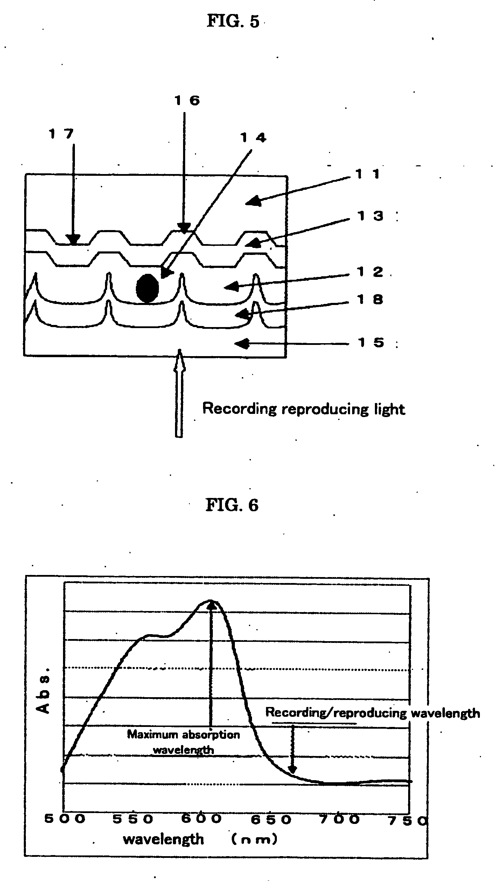

The absorption by a recording layer of light can be adjusted by way of the film thickness of the recording layer, the light absorption characteristics of the organic dye contained in the recording layer and so on. For an optical recording medium according to the present invention, the maximum absorption wavelength or the absorption peak wavelength of the organic dye (squarylium metal chelate compound expressed by the general formula (I)) is preferably between 580 nm to 630 nm as seen from FIG. 6. The maximum absorption wavelength or the absorption peak wavelength of the organic dye of the recording layer is preferably within the above range from the viewpoint of recording sensitivity and reflectance.

If necessary, the first recording layer and the second recording layer may additionally contain one or more other ingredients selected from polymer materials, stabilizers, dispersants, flame retardants, lubricants, antistatic agents, surfactants, plasticizers and so on.

Examples of polymer materials that can be used for the purpose of the present invention include ionomer resins, polyamide resins, vinyl resins, natural polymers, silicone, liquid rubber and so on. A silane coupling agent may be mixed and dispersed. Examples of stabilizers that can be used for the purpose of the present invention include transition metal complexes.

The first and second recording layers can be formed by any ordinary means such as evaporation, sputtering, CVD (chemical vapor deposition) or application of solution. They can be manufactured by a method of manufacturing an optical recording medium according to the invention as will be described hereinafter.

### [Substrate]

The substrate is required to be transparent relative to the laser beam to be used only when information is recorded and/or reproduced from the substrate side. It is not required to be transparent when information is recorded and/or reproduced from the recording layer side. According to the present invention, light with a recording wavelength of 580 nm to 720 nm is irradiated from the side of the surface of the first substrate. Therefore, only one of the substrates (the first substrate) is required to be transparent and the other substrate (the second substrate) may or may not be transparent.

There are no particular limitations to the material of the substrates and an appropriate material may be selected according to the application. Examples of materials that can be used for the substrates include plastic materials such as polyester resins, acrylic resins, polyamide resins, polycarbonate resins, polyolefin resins, phenol resins, epoxy resins and polyimide resins, glass, ceramics and metals.

The substrates are normally disk-shaped and it is preferable that the track pitch is normally 0.7 µm to 1.0 µm. The track pitch is 0.74 µm in the case of applications of DVD capacities.

The depth of the guide groove of the second substrate is preferably from 20 nm to 60 nm (200Å to 600Å). It is possible to suppress attenuation of reflected light due to the guide groove formed in the second substrate and achieve a high reflectance from the second recording layer with ease.

### [Reflective layer]

A material that shows a high reflectance relative to a laser beam is preferably used for the reflective layer. Examples of such materials include metals and semimetals such as Mg, Se, Y, Ti, Zr,' Hf, V, Nb, Ta, Cr, Mo, W, Mn, Re, Fe, Co, Ni, Ru, Rh, Pd, Ir, Pt, Cu, Ag, Au, Zn, Cd, Al, Ca, In, Si, Ge, Te, Pb, Po, Sn, Si, SiC and so on. Of these, Au, Al and Ag are particularly preferable because they provide a high reflectance. Any of these materials may be used alone or two or more of them may be combined for use.

The film thickness of the reflective layer is preferably normally from 5 nm to 300 nm (50Å to 3,000Å). It is more preferably from 5 nm to 30 nm (50Å to 300Å), most preferably from 10 nm to 15 nm (100Å to 150Å) when it is used as the translucent layer of the first recording layer.

When the first recording layer is a reflective film dedicated to reproduction or when the first recording layer is a recording layer that contains an organic dye and a reflective layer is formed further thereon, it is preferable to adjust the film thickness so as to make the transmittance of light equal to 40% or higher.

The transmittance of light hardly attenuates when the film thickness of the reflective layer is small. Therefore, the use of a thin film is preferable for the reflective layer. Particularly, when the reflective layer is formed by a material containing Ag as a principal ingredient, it is preferable to add Nd, Cu, Pd, In or some other similar metal by a small quantity to form an alloy in order to secure a desired level of durability with a film thickness within the above range. Such a metal is preferably added by about 0.2% by mass to 2% by mass in general.

There are no particular limitations to the film thickness of the reflective layer (the second reflective layer) and an appropriate film thickness may be selected according to the application, although a film thickness from 100, nm to 300 nm (1,000Å to 3,000Å) is preferable.

### [Protection layer]

The protection layer formed on the second recording layer also provides the effect of a barrier layer for protecting the recording layer from the intermediate layer. The two substrates can be bonded to each other with ease by using an adhesive agent that dissolves the organic dye for the intermediate layer.

Examples of materials that are preferably used for the protection layer include inorganic substances showing a high transmittance to light such as SiO, SiO₂, Si-N, MgF₂, SnO₂, SnS, ZnS and ZnS-SiO₂. Of these, the use of a material containing ZnS as a principal ingredient is particularly preferable because such a material is lowly crystalline and shows a high refractive index.

Examples of materials containing ZnS as a principal ingredient include ZnS-SiO₂ as well as ZnS-SiC, ZnS-Si, ZnS-Ge, ZnS-ZnO-GaN and ZnS-ZnO-In₂O₃-Ga₂O₃. The content ratio of ZnS in any of the above listed materials that contain ZnS as a principal ingredient is preferably between 60 mol% to 90 mol% from the viewpoint of crystallinity.

There are no particular limitations to the film thickness of the protection layer and an appropriate film thickness may be selected according to the application, although a film thickness close to the reflection peak is preferably selected when a material showing a high refractive index (n > 2) is used because a fluctuation peak of reflectance appears depending on the film thickness. More specifically, a film thickness of 5 nm to 30 nm, or of 80 nm to 180 nm is preferable in the case of a material that contains ZnS because otherwise it is not possible to obtain a satisfactory reflectance and a sufficient degree of recording signal modulation (contrast).

FIGS. 4 and 5 show the arrangement of the information layers of a one-side double-layer optical recording medium according to the present invention. FIG. 4 is a schematic cross sectional view of the first information layer of a one-side double-layer recording medium according to the present invention, showing the configuration thereof. Referring to FIG. 4, the recording mark forming section 4 that is formed in the first information layer (first dye recording layer 2) is arranged at a position in the substrate groove 6 of the first substrate 1. In the case of a one-side single-layer optical recording medium, the recording layer is the same as the first information layer of a one-side double-layer optical recording medium as pointed out above, the first information layer being bonded to a dummy substrate by means of an adhesive agent. Note that, the reference numbers 3, 5 and 7 respectively denote a first reflective layer, an intermediate layer, and a substrate inter-groove section, in FIG. 4.

FIG. 5 is a schematic cross sectional view of the second information layer of a one-side double-layer recording medium according to the present invention, showing the configuration thereof. The recording mark forming section 14 that is formed in the second information layer 12 is arranged at a position in the substrate inter-groove section 17 of the second substrate 11. Note that, the reference numbers 13, 15, 16, and 18 respectively denote a second reflective layer, a transparent intermediate layer, a substrate groove, and an inorganic protective layer, in FIG. 5.

As shown in FIGS. 4 and 5, the profile of the groove formed in the first substrate 1 and that of the groove formed in the second substrate 11 are not the same. For example, in the case of a DVD+R or a DVD-R with 4.7 GB and a 0.74 µm pitch, the depth (bottom width) of the groove formed in the first substrate 1 is preferably from 150 nm to 200 nm (1,500Å to 2,000Å) and from 0.1 µm to 0.35 µm respectively. This is because, when the film is formed by spin coating, the groove tends to be filled with dye so that the profile of the interface of the first recording layer 2 and the reflective layer 3 is determined by the extent to which the groove is filled with the applied liquid and the profile of the groove of the first substrate 1 and the above-cited ranges are suitable when interface reflection is utilized as seen from FIG. 4. On the other hand, the profile of the interface of the second recording layer 12 and the reflective layer 13 is determined by the profile of the groove of the second substrate 11 and hence the above-cited ranges are suitable when interface reflection is utilized as seen from FIG. 5.

With the recording/reproduction wavelength (about 650 nm) of DVD, the width (bottom width) of the groove formed in the second substrate is preferably from 0.1 µm and 0.35 µm. The reflectance can easily fall when the groove width is less than 0.1 µm, whereas it is difficult to uniformize the profiles of recording marks and jitters can increase with ease when the groove width exceeds 0.35 µm.

### [Substrate surface hard coat layer]

The substrate surface hard coat layer is formed for the purposes including (1) protection of the recording layer (reflection/absorption layer) against scars, dust and dirt, (2) improvement of the storage stability of the recording layer (reflection/absorption layer) and (3) improvement of the reflectance. To achieve these purposes, any of the inorganic materials and the organic materials listed for the undercoat layer may also be used for the hard coat layer.

Suitable examples of inorganic materials include SiO and SiO₂. Suitable examples of organic materials include polymethylacrylate resins, polycarbonate resins, epoxy resins, polystyrene resins, polyester resins, vinyl resins, cellulose, aliphatic hydrocarbon resins, aromatic hydrocarbon resins, natural rubber, styrene-butadiene resins, chloroprene rubber, wax, alkyd resins, drying oil, thermosoftening resins such as rosin, thermally melting resins and ultraviolet curing resins. Of these materials, ultraviolet curing resins can be manufactured with a high productivity and are most preferable for the substrate surface hard coat layer.

There are no particular limitations to the film thickness of the substrate surface hard coat layer and an appropriate thickness may be selected according to the application, although the thickness is preferably from 0.01 µm to 30 µm, more preferably from 0.05 µm to 10 µm.

### [Intermediate layer]

There are no particular limitations to the material of the intermediate layer so long as it is a transparent material that can bond the first information layer and the second information layer and an appropriate material may be selected according to the application. However, preferable materials that can be used for the intermediate layer include acrylate type, epoxy type and urethane type ultraviolet curing or thermosetting type adhesive agents and hot melt type adhesive agents.

There are no particular limitations to the film thickness of the intermediate layer and an appropriate film thickness may be selected according to the optical conditions of the recording/reproduction system, although a film thickness from 40 µm to 70 µm is preferable in case of DVD systems.

### [Undercoat layer]

An optical recording medium according to the present invention may include an undercoat layer as a component layer.

The undercoat layer is employed for the purposes including (1) improvement of adhesion, (2) provision of a barrier against water and/or gases, (3) improvement of the storage stability of the recording layers, (4) improvement of reflectance, (5) protection of the substrates and the recording layers against solvents and (6) formation of guide grooves, guide pits and pre-formats.

For the purpose (1) above, any of various polymer materials such as ionomer resins, polyamide resins, vinyl resins, natural resins, natural polymers, silicone, liquid rubber and so on and silane coupling agents may be used. For the purposes (2) and (3) above, any of various inorganic compounds such as SiO₂, MgF₂, SiO, TiO₂, ZnO, TiN, SiN and metals and semimetals such as Zn, Cu, Ni, Cr, Ge, Se, Au, Ag and Al may be used. For the purpose (4) above, any of metals such as Al and Ag and organic thin films showing a metallic luster such as a methine dye and a xanthene dye may be used. For the purposes (5) and (6) above, any of ultraviolet curing resins, thermosetting resins and thermoplastic resins may be used.

There are no particular limitations to the film thickness of the undercoat layer and an appropriate film thickness may be selected according to the application, although the film thickness is preferably from 0.01 µm to 30 µm, more preferably from 0.05 µm to 10 µm.

For the purpose of the present invention, the undercoat layer, the protection layer, the intermediate layer and the substrate surface hard coat layer may additionally contain one or more other ingredients selected from stabilizers, dispersants, flame retardants, lubricants, antistatic agents, surfactants, plasticizers and so on like the recording layers.

Now, a typical method for manufacturing an optical recording medium will be described below.

### [Optical recording medium manufacturing method - example]

A method for manufacturing a one-side double-layer optical recording medium according to the present invention at least includes a step of forming the first information layer, a step of forming the second information layer and a bounding step. If necessary, it may include some other step or steps.

### <First information layer forming step>

The first information layer forming step includes at least the first reflective layer forming step and the first recording layer forming step. If necessary, it may include some other step or steps. A substrate carrying the first recording layer may be prepared by way of steps similar to those for preparing conventional DVD+R and DVD-ROM.

### - First recording layer forming step -

In the first recording layer forming step, the first recording layer application liquid that contains an organic dye is applied onto the first substrate, on the surface of which at least one or more grooves or one or more pits are formed, and dried to produce the first recording layer. The organic dye preferably contains a squarylium metal chelate compound expressed by the general formula (I).

Techniques that can be used for applying the first recording layer application liquid include spinner application, spraying, roller coating, dipping and spin coating. Of these techniques, the use of spin coating is desirable because it can be used to control the thickness of the first recording layer by adjusting the concentration and the viscosity of the liquid and the drying temperature of the solvent for the first recording layer.

There are no particular limitations to the organic solvent that can be used for the application and an appropriate solvent may be selected according to the application. Examples of organic solvents include alcohols such as methanol, ethanol, isopropyl alcohol and 2,2,3,3-tetrafluoropropanol, ketones such as acetone, methyl ethyl ketone and cyclohexanone, amides such as N,N-dimethylformamide and N,N-dimethylacetoamide, sulfoxides such as dimethylsulfoxide, ethers such as tetrahydrofuran, dioxane, diethyl ether and ethylene glycol monomethyl ether, esters such as methyl acetate, ethyl acetate, aliphatic halogenated hydrocarbons such as chloroform, methylene chloride, dichloroethane, carbon tetrachloride and trichloroethane, aromatic compounds such as benzene, xylene, monochlorobenzene and dichlorobenzene, cellosolves such as methoxyethanol and ethoxyethanol, hydrocarbons such as hexane, pentane, cyclohexane and methylcyclohexane.

Preferably, the first recording layer is subjected to an annealing process in order to completely drive off the solvent remaining in the recording layer. The annealing temperature is from 60°C to 100°C.

### -First reflective layer forming step -

A reflective layer is formed on the first recording layer by a film forming means in the first reflective layer forming step. Film forming means that can be used for forming the first reflective layer include vacuum deposition, sputtering, plasma CVD, optical CVD, ion plating and electron beam evaporation, of which sputtering is preferable from the viewpoint of mass production and film quality.

### <Second information layer forming step>

The second information forming step includes at least the second reflective layer forming step, the second recording layer forming step and a protection layer forming step. If necessary, it may include some other step or steps. A substrate carrying the second recording layer may be prepared by way of steps similar to those for preparing conventional DVD+R and DVD-ROM.

### - Second reflective layer forming step -

In the second reflective layer forming step, a reflective layer is formed on the second substrate, on the surface of which at least one or more grooves or one or more pits are formed, by a film forming means.

Film forming means that can be used for forming the second reflective layer include vacuum deposition, sputtering, plasma CVD, optical CVD, ion plating and electron beam evaporation, of which sputtering is preferable from the viewpoint of mass production and film quality.

### - Second recording layer forming step -

In the second recording layer forming step, the second recording layer application liquid that contains an organic dye [expressed by the general formula (I)] is applied onto the reflective layer and dried to produce the second recording layer.

Techniques that can be used for applying the second recording layer application liquid are similar to those listed above for the first recording layer.

### - Protection layer forming step -

A protection layer is formed on the second recording layer in the protection layer forming step. Film forming means that can be used for forming the protection layer include vacuum deposition, sputtering, plasma CVD, optical CVD, ion plating and electron beam evaporation, of which sputtering is preferable from the viewpoint of mass production and film quality.

### <Bonding step>

In the bonding step, the first information layer and the second information layer are bonded to each other with the intermediate layer interposed between them in such a way that the first recording layer and the second recording layer are located inside.

An adhesive agent is dropped onto the surface of the second information layer where the protection layer is formed and the first information layer is made to cover the second information layer. At the same time, the adhesive agent is spread uniformly and cured by irradiating it with ultraviolet rays. The use of an ultraviolet curing adhesive agent is preferable because it shows a high transmittance relative to light.

The above-mentioned other steps include an undercoat layer forming step and a hard coat layer forming step.

### <Recording and reproduction method for optical recording medium>

A recording and reproduction method for an optical recording medium according to the present invention includes irradiating the optical recording medium with light having a wavelength of 580 nm to 720 nm from the side of the surface of the first substrate, so as to at least record signal information on or reproduce signal information from the first recording layer and the second recording layer.

More specifically, a recording light such as a semiconductor laser (e.g., having an oscillation wavelength of 650 nm) is emitted to the optical recording medium from the surface of the first substrate via an objective lens, while the optical recording medium is being driven to rotate at a predetermined linear velocity or a predetermined angular velocity. As a result of the irradiation of light, the first recording layer and the second recording layer absorb light to locally raise the temperature and generate pits so as to change the optical characteristics and consequently record information there. The information recorded in the above-described manner can be reproduced by irradiating a laser beam onto the optical recording medium, while driving the recording medium at a predetermined linear velocity, and detecting the reflected light.

### <Optical recording apparatus>

An optical recording apparatus according to the present invention is adapted to be mounted with an optical recording medium according to the present invention and has a unit which is configured to record information on and reproduce information from the optical recording medium.

While there are no particular limitations to an optical recording apparatus according to the present invention and may have any appropriate configuration, it typically has a laser beam source that may be a semiconductor laser for emitting a laser beam, a condenser lens for converging the laser beam emitted from the laser beam source onto the optical recording medium mounted on the spindle of the apparatus, a laser beam detector for detecting part of the laser beam emitted from the laser beam source and an optical element for guiding the laser beam emitted from the laser beam source to the condenser lens and the laser beam detector. If necessary, it may have one or more additional means.

The optical recording apparatus guides the laser beam emitted from the laser beam source to the condenser lens by means of the optical element and records information on the optical recording medium. At the same time, the optical recording apparatus guides a part of the laser beam emitted from the laser beam source to the laser beam detector and controls the quantity of light emitted from the laser beam source according to the amount of laser beam detected by the laser beam detector.

The laser beam detector converts the amount of laser beam it detects into a voltage or an electric current and outputs it as signal representing the detected amount.

The other means as mentioned above include a control means. There are no particular limitations to the control means so long as it can control the operations of the various means of the optical recording apparatus and an appropriate control means may be selected according to the application. Examples of control means that can be used for the purpose of the present invention include sequencers and computers.

Since an optical recording apparatus according to the present invention is adapted to be mounted by an optical recording medium according to the present invention that provides good recording signal characteristics and suppresses the amount of cross talks of recording marks; it can stably record signals with a high reflectance and a high percentage modulation.

### [Examples]

Now, the present invention will be described further by way of examples, although the present invention is by no means limited to the examples.

Each of Example 1 through Example 6 below is for synthesis of a squarylium compound forming a metal complex according to the present invention, whereas each of Example 7 through Example 19, Comparative Example 1 and Comparative Example 2 is for evaluation of an optical recording medium.

### (Example 1)

15.5 g of benzylacetone and 12.2 g of 1-methyl-1-phenylhydrazine were dissolved into 30 ml of butanol. After one hour at 60°C, to the solution were added 4.9 g of sulfuric acid. After four hours at 100°C, 30 ml of water were added and the insoluble was filtered. A solution of the obtained solid in 25 ml of isopropanol and 14.9 g of benzyl bromide was treated at 80°C for 5 hours. After filtering the produced solid, 7 ml of toluene and 10 ml of a 10% aqueous sodium hydroxide were added to the obtained solid and 14 ml of a butanol solution containing 6.49 g of 3-hydroxy-4-(5-hydroxy-1-phenyl-3-trifluoromethylpyrazole-4-yl)cyclobut ene-1,2-dione was added to the organic layer and treated at 110°C for 20 hours. Subsequently, 9.90 g of the compound [1] was obtained by filtering the deposit. 12 ml of toluene, 12 ml of ethanol, 0.10 g of acetic acid and 0.66 g of aluminumtris(ethylacetoacetate) were added to 3.0 g of the compound [1] and made to react at 80°C for 3 hours. Thus, 1.10 g of the compound 1-Al was obtained by filtering the insoluble. The result of NMR analysis is shown below.
¹H NMR δ (CDCl₃) ppm: 3.00 (9H s), 3.06-3.26 (6H, m), 4.72-4.95 (6H, m), 6.16 (3H, m), 6.19-6.22 (6H, m), 6.27-6.36 (6H, m), 6.46-6.53 (3H, m), 6.62-6.76 (9H, m), 6.86-7.22 (27H, m), 7.52-7.64 (6H, m).

### (Example 2)

The process of Example 1 was followed except that 1-methyl-1-pehnyhydrazine was replaced by 1-benzyl-1-phenylhydrazine to obtain 10.66 g of the compound [4]. Thereafter, the process of Example 1 was also followed except that the compound [1] was replaced by the compound [4] to obtain 1.20 g of the compound 4-Al. Some of the results of an analysis by NMR are listed below.
¹H NMR δ (CDCl₃) ppm: 2.83-4.95 (21H, m), 5.63-7.97 (72H, m).

### (Example 3)

35 ml of butanol were added to 10.4 g of benzylacetone and 11.1 g of p-tolylhydrazine hydrochloride. After agitating the solution at 100°C for 4 hours, 30 ml of water were added and the insoluble was filtered. 100 g of dimethylsulfoxide and 9.90 g of potassium t-butoxide were added to the obtained solid and treated at 30°C for 30 minutes and subsequently 11.8 g of methyl iodide was added. After agitating for 1.5 hours, a 3N aqueous solution of hydrochloric acid was added and the insoluble was filtered. Then, 80 ml of isopropanol and 8.27 g of benzyl bromide were added to the obtained solid and treated at 80°C for 8 hours. After the reaction, 80 ml of toluene was added and the deposit was filtered. Then, 9 ml of toluene and 9 ml of a 10% aqueous solution of sodium hydroxide were added to the obtained solid and 18 ml of butanol solution containing 8.10 g of 3-hydroxy-4-(5-hydroxy-1-phenyl-3-trifluoromethylpyrazole-4-il)cyclobute ne-1,2-dione were added to the organic layer and treated at 100°C for 4 hours. Subsequently, 2.49 g of the compound [6] were obtained by filtering the deposit. Thereafter, the process of Example 1 was followed except that the compound [1] was replaced by the compound [6] to obtain 1.59 g of the compound 6-Al. Some of the results of an analysis by NMR are listed below.
¹H NMR δ (CDCl₃₎ ppm: 2.29-2.45 (9H, m), 2.97-4.72 (21H, m), 5.75-6.59 (3H, m), 6.62-8.21 (54H, m).

### (Example 4)

The process of Example 1 was followed except that 1-methyl-1-pehnyhydrazine was replaced by 1-ethyl-1-p-tolylhydrazine to obtain 9.85 g of the compound [7]. Thereafter, the process of Example 1 was also followed except that the compound [1] was replaced by the compound [7] to obtain 2.08 g of the compound 7-Al. Some of the results of an analysis by NMR are listed below.
¹H NMR δ (CDCl₃) ppm: 0.37-0.67 (9H, m), 2.32-2.49 (9H, m), 2.90-2.68 (18H, m), 5.45-8.43 (57H, m).

### (Example 5)

The process of Example 4 was followed except that 3-hydroxy-4-(5-hydroxy-1-phenyl-3-trifluoromethylpyrazole-4-il)cyclobute ne-1,2-dione was replaced by 3-hydroxy-4-(3-isopropyl-5-hydroxy-1-phenylpyrazole-4-il)cyclobutene-1,2 -dione to obtain 10.89 g of the compound [9]. Thereafter, the process of Example 4 was followed except that the compound [1] was replaced by the compound [9] to obtain 2.08 g of the compound 9-Al. Some of the results of an analysis by NMR are listed below.
¹H NMR δ (CDCl₃) ppm: 0.37-0.67 (9H, m), 2.32-2.49 (9H, m), 2.90-4.68 (18H, m), 5.45-7.69 (51H, m), 7.91-8.43 (6H, m).

### (Example 6)

The process of Example 3 was followed except that p-tolylhydrazine hydrochloride was replaced by p-chlorophenylhyrazine hydrochloride, to thereby obtain 4.19 g of the compound [10]. Thereafter, the process of Example 1 was followed expect that the compound [1] was replaced with the compound [10], to thereby obtain 3.39 g of the compound 10-Al. Some of the results of an analysis by NMR are listed below.
¹H NMR δ (CDCl₃) ppm: 2.90 (3H, d, J = 13.6 Hz), 3.01 (9H, s), 3.14 (3H, d, J = 13.6 Hz), 4.72 (3H, d, J = 13.6 Hz), 4.77 (3H, d, J = 13.6 Hz), 6.00-6.02 (6H, m), 6.27 (3H, s), 6.27-6.29 (6H, m), 6.39-6.42 (3H, m), 6.75-6.79 (6H, m), 6.96-7.16 (27H, m), 7.55-7.57 (6H, m).

### (Example 7)

A second substrate made of polycarbonate resins and having a guide groove undulation pattern with a depth of 33 nm, a groove width (bottom width) of 0.25 µm and a track pitch of 0.74 µm, a diameter of 120 mm and a thickness of 0.57 mm was prepared. A second reflective layer of AgIn (Ag/In = 99.5/0.5) was formed on the second substrate to a thickness of about 140 nm by sputtering, using Ar gas as sputtering gas.

Then, a second recording layer was formed to a thickness of about 80 nm by applying an application liquid, which was obtained by dissolving a squarylium metal chelate compound expressed by the compound No. 12-A1 in Table 2 into 2,2,3,3-tetrafluoropropanol, to the surface of the AgIn reflective layer by spin coating.

Thereafter, a protection layer of ZnS-SiC (mol ratio: ZnS/SiC = 8/2) was formed to a thickness of 15 nm on the second recording layer by sputtering, using Ar gas as sputtering gas. As a result, a second information layer was prepared.

Meanwhile, DVD-ROM information pits were formed on the surface of a first substrate having a diameter of 120 mm and a thickness of 0.57 mm and made of polycarbonate resin. Then, a reflective layer of AgIn (about 99.5/0.5) was formed to a thickness of 10 nm on the first substrate. As a result, a first information layer was prepared.

Then, the first information layer and the second information layer were bonded to each other by means of an ultraviolet curing type adhesive agent (KAYARAD DVD576: tradename, available from Nippon Kayaku Co., Ltd.) to produce a double-layer type optical recording medium having a layer arrangement as shown in FIG. 2.

DVD (8-16) signals were recorded on the second recording layer of the obtained optical recording medium under the conditions of wavelength: 659 nm, NA: 0.65 and linear velocity: 45.96 m/s (12 times faster) and the signal reproduction was evaluated under the DVD-ROM replay conditions of wavelength: 650 nm, NA: 0.60 and linear velocity: 3.83 m/s to obtain results including reflectance after recording (I14H): 23%, percentage modulation (I14/I14H): 63%, Jitter: 8.4% and PIsum8 (PI error): 50 or less, which satisfy the requirements of the DVD+R Standard. The recording evaluation apparatus that was used was ODU1000 (tradename, available from Palstec). The optical recording medium was irradiated with light by means of a 50,000 lux xenon lamp from the recording/reproduction side for 100 hours and subsequently PIsum8 was observed to find that PIsum8 was 50 or less to prove that the optical recording medium has an excellent resistance against light. Table 4 below summarizes the obtained results.

### (Example 8)

In a similar manner to that of Example 7, a second information layer was formed on a second substrate made of polycarbonate resins and having a guide groove undulation pattern with a depth of 33 nm, a groove width (bottom wide) of 0.25 µm and a track pitch of 0.74 µm, a diameter of 120 mm and a thickness of 0.57 mm.

Meanwhile, a first substrate made of polycarbonate resins and having a guide groove undulation pattern with a depth of 160 nm, a groove width (bottom wide) of 0.25 µm and a track pitch of 0.74 µm, a diameter of 120 mm and a thickness of 0.58 mm was prepared. A squarylium metal chelate compound expressed by the compound No. 12-A1 in Table 2 and a formazan metal chelate compound expressed by the structural formula (3) below were mixed to a mass ratio (squarylium metal chelate compound / formazan metal chelate compound) = 7/3 and dissolved into 2,2,3,3-tetrafluoropropanol to prepare an application liquid. Then, the application liquid was applied to the first substrate by spin coating to form a first recording layer having a thickness of 50 nm. The formazan metal chelate compound was used for the mixture as a light-resistant auxiliary agent.

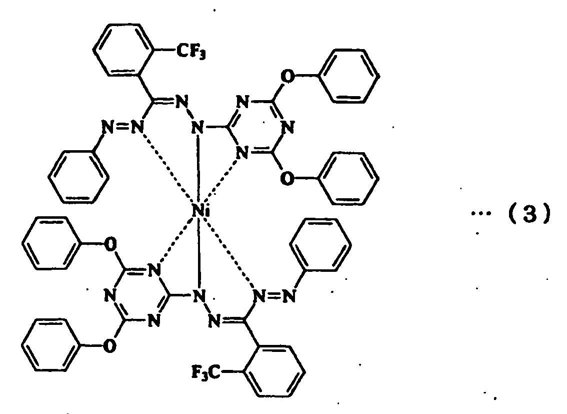

Then, a 10 nm-thick reflective layer of AgIn (99.5/0.5) was formed on the first recording layer by sputtering, using Ar gas as sputtering gas. As a result, a first information layer was prepared.

Then, the first information layer and the second information layer were bonded to each other by means of an ultraviolet curing type adhesive agent (KAYARAD DVD576: tradename, available from Nippon Kayaku Co., Ltd.) to produce a double-layer type optical recording medium having a layer arrangement as shown in FIG. 3.

The first recording layer and the second recording layer of the obtained optical recording medium were evaluated as in Example 7. Both the first recording layer and the second recording layer produced results that satisfy the requirements of the DVD+R Standard.

### <First recording layer>

reflectance after recording (I14H): 18%, percentage modulation
(I14/I14H): 65%, Jitter: 7.8%, PIsum8: 50 or less.

### <Second recording layer>

reflectance after recording (I14H): 17%, percentage modulation
(I14/I14H): 62%, Jitter: 8.5%, PIsum8: 50 or less.

The optical recording medium was irradiated with light by means of a 50,000 lux xenon lamp from the recording/reproduction side for 100 hours and subsequently PIsum8 was observed and found that PIsum8 of the first recording layer was 100 or less, and PIsum8 of the second recording layer was 50 or less. Table 4 below summarizes the obtained results.

### (Example 9)

The process of Example 8 was followed except that the squarylium metal chelate compound in the second recording layer was replaced by the compound No. 13-Al in Table 2 to prepare an optical recording medium.

The obtained optical recording medium was evaluated as in Example 8. As a result, it was confirmed that the optical recording medium had signal characteristics that satisfy the requirements of the DVD+R Standard and an excellent resistance against light. Table 4 below summarizes the obtained results.

### (Example 10)

The process of Example 8 was followed except that the squarylium metal chelate compound in the second recording layer was replaced by a mixture of the compound No. 7-Al in Table 1 and the compound No. 13-Al in Table 2 with a mass ratio (No. 7-Al / No. 13-Al) = 7/3 to prepare an optical recording medium.

The obtained optical recording medium was evaluated as in Example 8. As a result, it was confirmed that the optical recording medium had signal characteristics that satisfy the requirements of the DVD+R Standard and an excellent resistance against light. Table 4 below summarizes the obtained results.

### (Example 11)

The process of Example 10 was followed except that the squarylium metal chelate compound in the second recording layer was replaced by a mixture of the compound No. 14-Al and the compound No. 12-Al in Table 2 with a mass ratio (No. 14-Al / No. 12-Al) = 6/4 to prepare an optical recording medium.

The obtained optical recording medium was evaluated as in Example 10. As a result, it was confirmed that the optical recording medium had signal characteristics that satisfy the requirements of the DVD+R Standard and an excellent resistance against light. Table 4 below summarizes the obtained results.

### (Example 12)

The process of Example 10 was followed except that the squarylium metal chelate compound in the second recording layer was replaced by a mixture of a squarylium metal chelate compound expressed as the compound No. 3-Al in Table 1 and a cyanine dye expressed by the structural formula (4) below with a mass ratio (squarylium metal chelate compound / cyanine dye) = 6/4 weight ratio. The cyanine dye was used for the mixture as a light absorption regulating agent. 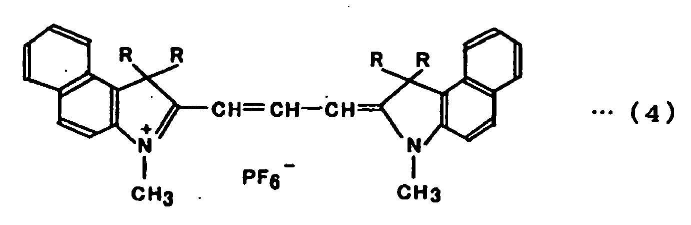 (In the above formula, R independently represents CH₃ or a benzyl group and at least two of the Rs are benzyl groups.)

The obtained optical recording medium was evaluated as in Example 10. As a result, it was confirmed that the optical recording medium had signal characteristics that satisfy the requirements of the DVD+R Standard and an excellent resistance against light. Table 4 below summarizes the obtained results.

### (Examples 13 through 17)

In these examples, the process of Example 12 was followed except that the squarylium metal chelate compound in the second recording layer was replaced respectively by squarylium metal chelate compounds expressed as the compound No. 1-Al, the compound No: 2-Al, the compound No. 4-Al and the compound No. 5-Al in Table 1 and compound No. 19-Al in Table 3 to prepare optical recording mediums.

The obtained optical recording mediums were evaluated as in Example 12. As a result, it was confirmed that the optical recording mediums had signal characteristics that satisfy the requirements of the DVD+R Standard and an excellent resistance against light. Table 4 below summarizes the obtained results.

### (Comparative Example 1)

The process of Example 14 was followed except that the squarylium metal chelate compound in the second recording layer was replaced by a squarylium compound (not a metal chelate compound) expressed by the structural formula (5) below to prepare an optical recording medium.

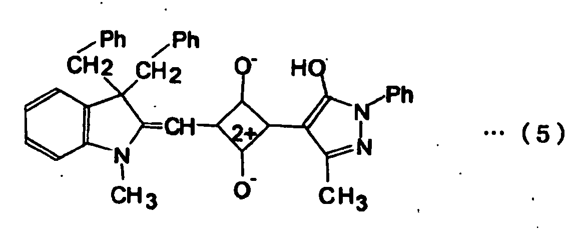

The obtained optical recording medium was evaluated as in Example 14. As a result, it was confirmed that the optical recording medium had jitter characteristics that did not satisfy the requirements of the DVD+R Standard. Table 4 below summarizes the obtained results.

### (Comparative Example 2)

The process of Example 14 was followed except that the squarylium metal chelate compound in the second recording layer was replaced by an Al chelate compound expressed by the structural formula (6) below to prepare an optical recording medium.

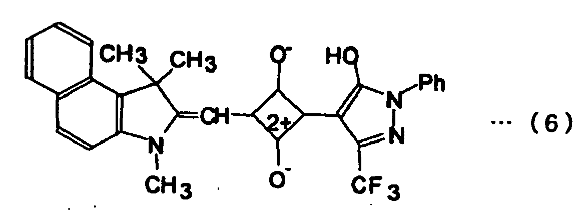

The obtained optical recording medium was evaluated as in Example 14. As a result, it was confirmed that the optical recording medium had jitter characteristics that did not satisfy the requirements of the DVD+R Standard. Table 4 below summarizes the obtained results.

**Table 4**

| Example Comp. ex. | Squarylium metal chelate compound | Recording layer | 114H | 114/114H | Jitter | PIsum8 | |
|---|---|---|---|---|---|---|---|
| | | | (%) | (%) | (%) | Initial | After exposure to light |
| Example 7 | No. 12-Al | 2^{nd} according layer | 23 | 63 | 8.4 | 50 or less | 50 or less |
| Example 8 | No. 12-Al | 1st recording layer | 18 | 65 | 7.8 | 50 or less | 100 or less |
| Example 8 | No. 12-Al | 2^{nd} recording layer | 17 | 62 | 8.5 | 50 or less | 50 or less |
| Example 9 | No. 13-Al | 2^{nd} recording layer | 18 | 63 | 7.9 | 50 or less | 50 or less |
| Example 10 | No. 7-Al/ No. 13-Al | 2^{nd} recording layer | 20 | 64 | 8.0 | 50 or less | 50 or less |
| Example 11 | No. 14-Al/ No. 12-Al | 2^{nd} recording layer | 19 | 62 | 8.7 | 50 or less | 50 or less |
| Example 12 | No. 3-Al | 2^{nd} recording layer | 22 | 62 | 8.4 | 50 or less | 50 or less |
| Example 13 | No. 1-Al | 2^{nd} recording layer | 21 | 60 | 8.5 | 50 or less | 50 or less |
| Example 14 | No. 2-Al | 2^{nd} recording layer | 23 | 62 | 8.8 | 50 or less | 50 or less |
| Example 15 | No. 4-Al | 2^{nd} recording layer | 21 | 62 | 7.9 | 50 or less | 50 or less |
| Example 16 | No. 5-Al | 2^{nd} recording layer | 17 | 61 | 8.5 | 50 or less | 50 or less |
| Example 17 | No. 19-Al | 2^{nd} recording layer | 22 | 64 | 8.4 | 50 or less | 50 or less |
| Comp. ex. 1 | V | 2^{nd} recording layer | 20 | 60 | 11.5 | 300 or more | - |
| Comp. ex. 2 | VI-Al | 2^{nd} recording layer | 16 | 65 | 11.1 | 300 or more | - |
| DVD+R standard value | | | 16 or more | 60 or more | 9 or less | 280 or less | 280 or less |

### (Example 18)

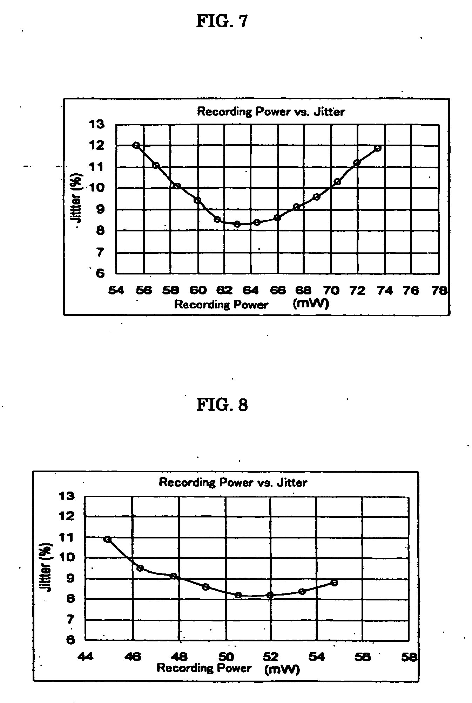

DVD (8-16) signals were recorded on the optical recording medium obtained in Example 13 under the conditions of wavelength: 659 nm, NA: 0.65 and linear velocity: 61.28 m/s (16 times faster) and the signal reproduction was evaluated under the DVD-ROM replay conditions of wavelength: 650 nm, NA: 0.60 and linear velocity: 3.83 m/s to evaluate the recording power dependency of jitter. As a result; it was confirmed that the optical recording medium had Jitter characteristics that satisfy the requirements of the DVD+R standard. FIG. 7 summarizes the obtained results.

### (Example 19)

A first substrate made of polycarbonate resins and having a guide groove undulation pattern with a depth of 160 nm, a groove width (bottom width) of 0.25 µm and a track pitch of 0.74 µm, a diameter of 120 mm and a thickness of 0.60 mm was prepared. A squarylium metal chelate compound expressed by the compound No. 1-Al in Table 1, a formazan metal chelate compound expressed by the structural formula (3) below and a cyanine dye expressed by the structural formula (4) below were mixed to a mass ratio (squarylium metal chelate compound / formazan metal chelate compound / cyanine dye) = 5/2/3 on the first substrate and an application liquid dissolving 2,2,3,3-tetrafluoropropanol was applied by spin coating to form a first recording layer having a thickness of about 45 nm. 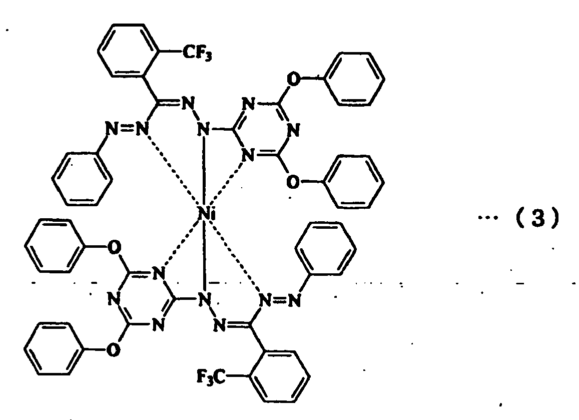 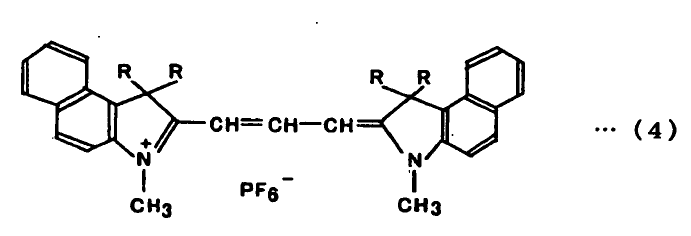 (In the above formula (4), R independently represents CH₃ or a benzyl group and at least two of the Rs are benzyl groups.)

Then, a reflective layer of AgIn (Ag/In = 99.5/0.5) was formed on the first substrate to a thickness of about 100 nm by sputtering, using Ar gas as sputtering gas.

Thereafter, the above first information layer was bonded to a polycarbonate dummy substrate having the same profile by means of an ultraviolet curing type adhesive agent (KAYARAD DVD576: tradename, available from Nippon Kayaku Co., Ltd.) to produce a one-side single-layer type optical recording medium.

DVD (8-16) signals were recorded on the thus obtained optical recording medium under the conditions of wavelength: 659 nm, NA: 0.65 and linear velocity: 62.82 m/s (18 times faster) and the signal reproduction was evaluated under the DVD-ROM replay conditions of wavelength: 650 nm, NA: 0.60 and linear velocity: 3.49 m/s to evaluate the recording power dependency of jitter. As a result, it was confirmed that the optical recording medium had signal characteristics that satisfy the requirements of the DVD+R Standard. FIG. 8 summarizes the obtained results.

Thus, it will be seen from the above results of evaluation, an optical recording medium according to the present invention provides excellent recording signal characteristics and can suitably be used for recordable DVD systems. Particularly, it can find broad applications as DVD+R and DVD-R.

## Claims

1. A squarylium compound forming a metal complex,
wherein the squarylium compound is expressed by the general formula (I) below: 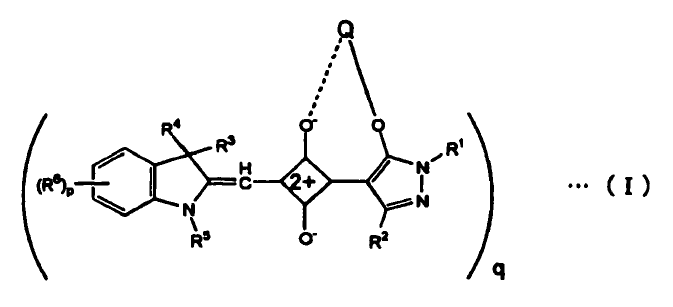 where R¹ is a phenyl group and R² is a trifluoromethyl group or an alkyl group having a branched chain; Q represents a metal atom having coordinating ability; q represents 2 or 3; R³ represents a substituted or unsubstituted aralkyl group, or a substituted or unsubstituted aryl group; R⁴ represents a substituted or unsubstituted alkyl group, a substituted or unsubstituted aralkyl group, or a substituted or unsubstituted aryl group; R⁵ represents a hydrogen atom, a substituted or unsubstituted alkyl group, a substituted or unsubstituted aralkyl group, or a substituted or unsubstituted aryl group; R⁶ represents a halogen atom, a substituted or unsubstituted alkyl group, a substituted or unsubstituted aralkyl group, a substituted or unsubstituted aryl group, a nitro group, a cyano group, or a substituted or unsubstituted alkoxy group; p represents any integer ranging from 0 to 4; and R⁶s are the same or different to each other and any two adjacently located R⁶s optionally form a substituted or unsubstituted aromatic ring together with two adjacent carbon atoms when p is 2, 3 or 4.

2. The squarylium compound according to claim 1, wherein Q is aluminum.

3. The squarylium compound according to any of claim 1 and 2, wherein R² is an isopropyl group.

4. The squarylium compound according to any of claims 1 to 3, wherein R³ and R⁴ are the same or different and each of R³ and R⁴ is a substituted or unsubstituted benzyl group, or a substituted or unsubstituted phenyl group.

5. The squarylium compound according to claim 4, wherein each of R³ and R⁴ is a benzyl group.

6. The squarylium compound according to claim 4, wherein R³ is a phenyl group and R⁴ is a benzyl group.

7. The squarylium compound according to any of claims 1 to 6, wherein R⁵ is a benzyl group or an ethyl group.

8. An optical recording medium comprising:
a substrate; and
a recording layer disposed on the substrate, wherein the recording layer comprises at least the squarylium compound forming a metal complex as defined in any of claims 1 to 7.

9. The optical recording medium according to claim 8, wherein the recording layer further comprises a squarylium compound forming a metal complex, wherein the squarylium compound is expressed by the general formula (II) below: 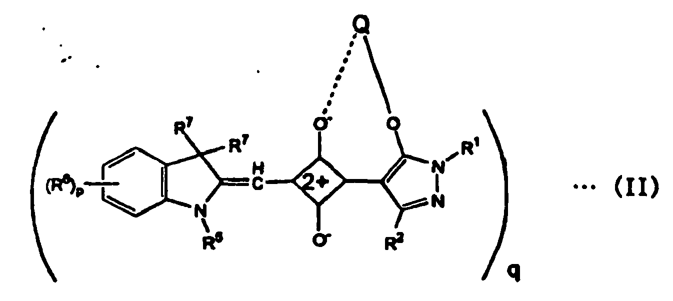 where R¹ and R² are the same or different to each other and each of R¹ and R² represents a substituted or unsubstituted alkyl group, a substituted or unsubstituted aralkyl group, a substituted or unsubstituted aryl group, or a substituted or unsubstituted heterocyclic group; Q represents a metal atom having coordinating ability; q represents 2 or 3; R⁷ represents a substituted or unsubstituted alkyl group, or two R⁷s are optionally bonded to each other to form either an alicyclic hydrocarbon ring or heterocyclic ring; R⁵ represents a hydrogen atom, a substituted or unsubstituted alkyl group, a substituted or unsubstituted aralkyl group, or a substituted or unsubstituted aryl group; R⁶ represents a halogen atom, a substituted or unsubstituted alkyl group, a substituted or unsubstituted aralkyl group, a substituted or unsubstituted aryl group, a nitro group, a cyano group, or a substituted or unsubstituted alkoxy group; p represents any integer ranging from 0 to 4; and R⁶s are the same or different to each other and any two adjacently located R⁶s optionally form a substituted or unsubstituted aromatic ring together with two adjacent carbon atoms when p is 2, 3 or 4.

10. The optical recording medium according to claim 8 or 9, wherein the recording layer further comprises a formazan metal chelate forming a metal complex and a formazan compound expressed by either the general formula (III) or (IV) below: 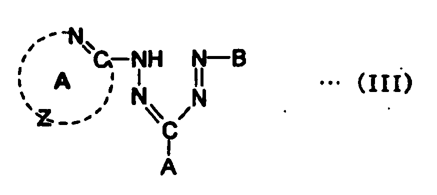 where ring A represents a substituted or unsubstituted 5-membered or 6-membered ring containing a nitrogen atom; Z represents an atom group forming the ring A wherein the nitrogen-containing heterocyclic ring is optionally condensed with some other ring; A represents a substituted or unsubstituted alkyl group, a substituted or unsubstituted aryl group, a substituted or unsubstituted alkylcarbonyl group, a substituted or unsubstituted arylcarbonyl group, a substituted or unsubstituted alkenyl group, a substituted or unsubstituted heterocyclic ring residue, or a substituted or unsubstituted alkyloxycarbonyl group; and B represents a substituted or unsubstituted alkyl group, a substituted or unsubstituted alkenyl group, or a substituted or unsubstituted aryl group, 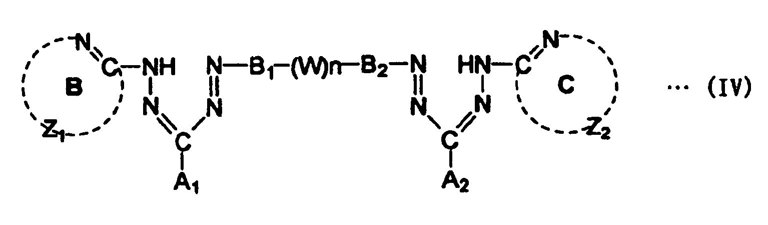 where ring B and ring C are the same or different to each other and each of ring B and ring C represents a substituted or unsubstituted 5-membered or 6-membered ring containing a nitrogen atom; Z₁ and Z₂ respectively represent atom groups forming the ring B and the ring C, and each of the nitrogen-containing heterocyclic rings is optionally condensed with some other ring; each of A₁ and A₂ represents a substituted or unsubstituted alkyl group, a substituted or unsubstituted aryl group, a substituted or unsubstituted alkylcarbonyl group, a substituted or unsubstituted arylcarbonyl group, a substituted or unsubstituted alkenyl group, a substituted or unsubstituted heterocyclic ring residue, or a substituted or unsubstituted alkyloxycarbonyl group; each of B₁ and B₂ represents a substituted or unsubstituted alkylene group, a substituted or unsubstituted alkenylene group, or a substituted or unsubstituted arylene group; W represents -CH₂- or -SO₂-; and n represents an integer of 0 or 1.

11. The optical recording medium according to any of claims 8 to 10, wherein the recording layer further comprises a cyanine dye being expressed by the following structural formula (4): 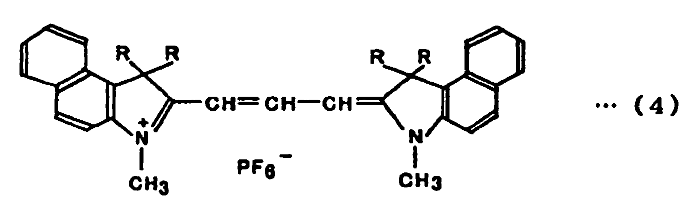 where each of Rs represents -CH₃ or a benzyl group, and at least two Rs are benzyl groups.

12. A recording and reproduction method of an optical recording medium, comprising:
irradiating the optical recording medium as defined in any of claims 8 to 11 with a light having a recording wavelength of 580 nm to 720 nm from the side of the substrate of the optical recording medium so as to perform either recording or reproducing of signal information.

## Patentansprüche

1. Squaryliumverbindung, die einen Metallkomplex bildet,
wobei die Squaryliumverbindung durch die nachstehende allgemeine Formel (I) ausgedrückt wird: 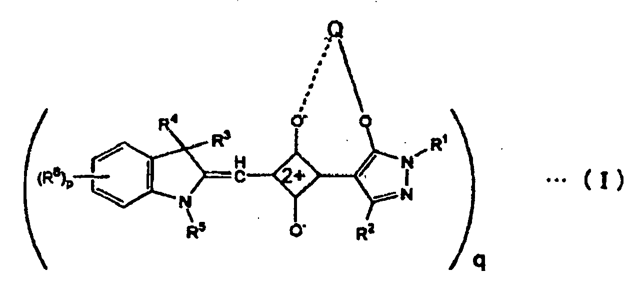 worin R¹ eine Phenylgruppe ist und R² eine Trifluormethylgruppe oder eine Alkylgruppe mit einer verzweigten Kette ist; Q ein Metallatom mit Koordinierungsfähigkeit darstellt; q 2 oder 3 darstellt; R³ eine substituierte oder unsubstituierte Aralkylgruppe oder eine substituierte oder unsubstituierte Arylgruppe darstellt; R⁴ eine substituierte oder unsubstituierte Alkylgruppe, eine substituierte oder unsubstituierte Aralkylgruppe oder eine substituierte oder unsubstituierte Arylgruppe darstellt; R⁵ ein Wasserstoffatom, eine substituierte oder unsubstituierte Alkylgruppe, eine substituierte oder unsubstituierte Aralkylgruppe oder eine substituierte oder unsubstituierte Arylgruppe darstellt; R⁶ ein Halogenatom, eine substituierte oder unsubstituierte Alkylgruppe, eine substituierte oder unsubstituierte Aralkylgruppe, eine substituierte oder unsubstituierte Arylgruppe, eine Nitrogruppe, eine Cyanogruppe oder eine substituierte oder unsubstituierte Alkoxygruppe darstellt; p eine ganze Zahl im Bereich von 0 bis 4 darstellt; und die R⁶ gleich oder verschieden voneinander sind und irgendwelche zwei zueinander benachbarte R⁶
wahlweise zusammen mit zwei benachbarten Kohlenstoffatomen einen substituierten oder unsubstituierten aromatischen Ring bilden, wenn p 2, 3 oder 4 ist.

2. Squaryliumverbindung gemäß Anspruch 1, wobei Q Aluminium ist.

3. Squaryliumverbindung gemäß irgendeinem aus den Ansprüchen 1 und 2, wobei R² eine Isopropylgruppe ist.

4. Squaryliumverbindung gemäß irgendeinem aus den Ansprüchen 1 bis 3, wobei R³ und R⁴ gleich oder verschieden sind und jedes aus R³ und R⁴ eine substituierte oder unsubstituierte Benzylgruppe oder eine substituierte oder unsubstituierte Phenylgruppe ist.

5. Squaryliumverbindung gemäß Anspruch 4, wobei jedes aus R³ und R⁴ eine eine Benzylgruppe ist.

6. Squaryliumverbindung gemäß Anspruch 4, wobei R³ eine Phenylgruppe ist und R⁴ eine Benzylgruppe ist.

7. Squaryliumverbindung gemäß irgendeinem aus den Ansprüchen 1 bis 6, wobei R⁵ eine Benzylgruppe oder eine Ethylgruppe ist.

8. Optisches Aufzeichnungsmedium, umfassend:
ein Substrat; und
eine auf dem Substrat angeordnete Aufzeichnungsschicht, wobei die Aufzeichnungsschicht mindestens die einen Metallkomplex bildende Squaryliumverbindung wie in irgendeinem der Ansprüche 1 bis 7 definiert umfasst.

9. Optisches Aufzeichnungsmedium gemäß Anspruch 8, wobei die Aufzeichnungsschicht ferner eine einen Metallkomplex bildende Squaryliumverbindung umfasst, wobei die Squaryliumverbindung durch die nachstehende allgemeine Formel (II) ausgedrückt wird: 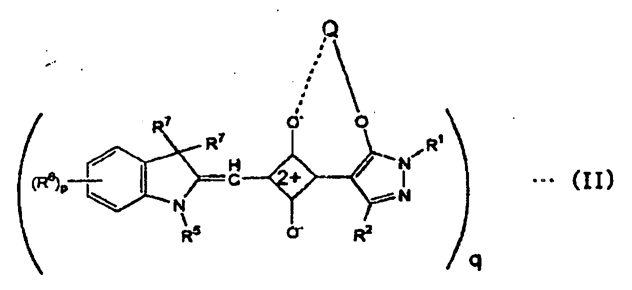 worin R¹ und R² gleich oder voneinander verschieden sind und jedes aus R¹ und R² eine substituierte oder unsubstituierte Alkylgruppe, eine substituierte oder unsubstituierte Aralkylgruppe, eine substituierte oder unsubstituierte Arylgruppe oder eine substituierte oder unsubstituierte heterocyclische Gruppe darstellt; Q ein Metallatom mit Koordinierungsfähigkeit darstellt; q 2 oder 3 darstellt; R⁷ eine substituierte oder unsubstituierte Alkylgruppe darstellt oder zwei R⁷ wahlweise miteinander verbunden sind, um entweder einen alicyclischen Kohlenwasserstoffring oder einen heterocyclischen Ring zu bilden; R⁵ ein Wasserstoffatom, eine substituierte oder unsubstituierte Alkylgruppe, eine substituierte oder unsubstituierte Aralkylgruppe oder eine substituierte oder unsubstituierte Arylgruppe darstellt; R⁶ ein Halogenatom, eine substituierte oder unsubstituierte Alkylgruppe, eine substituierte oder unsubstituierte Aralkylgruppe, eine substituierte oder unsubstituierte Arylgruppe, eine Nitrogruppe, eine Cyanogruppe oder eine substituierte oder unsubstituierte Alkoxygruppe darstellt; p eine ganze Zahl im Bereich von 0 bis 4 darstellt; und die R⁶ gleich oder verschieden voneinander sind und irgendwelche zwei zueinander benachbarte R⁶ wahlweise zusammen mit zwei benachbarten Kohlenstoffatomen einen substituierten oder unsubstituierten aromatischen Ring bilden, wenn p 2, 3 oder 4 ist.

10. Optisches Aufzeichnungsmedium gemäß Anspruch 8 oder 9, wobei die Aufzeichnungsschicht ferner ein einen Metallkomplex bildendes Formazan-Metallchelat und eine durch entweder die nachstehende allgemeine Formel (III) oder (IV) ausgedrückte Formazanverbindung umfasst: 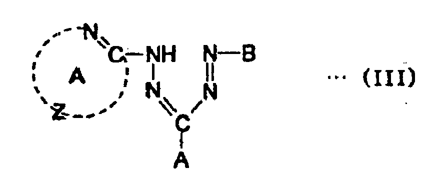 wobei Ring A einen substituierten oder unsubstituierten, ein Stickstoffatom enthaltenden 5-gliedrigen oder 6-gliedrigen Ring darstellt; Z eine den Ring A bildende Atomgruppe darstellt, wobei der Stickstoff-haltige heterocyclische Ring wahlweise mit irgendeinem anderen Ring kondensiert ist; A eine substituierte oder unsubstituierte Alkylgruppe, eine substituierte oder unsubstituierte Arylgruppe, eine substituierte oder unsubstituierte Alkylcarbonylgruppe, eine substituierte oder unsubstituierte Arylcarbonylgruppe, eine substituierte oder unsubstituierte Alkenylgruppe, einen substituierten oder unsubstituierten heterocyclischen Ringrest oder eine substituierte oder unsubstituierte Alkyloxycarbonylgruppe darstellt; und B eine substituierte oder unsubstituierte Alkylgruppe, eine substituierte oder unsubstituierte Alkenylgruppe oder eine substituierte oder unsubstituierte Arylgruppe darstellt, 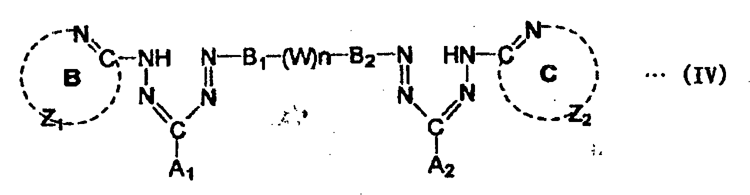 wobei Ring B und Ring C gleich oder voneinander verschieden sind, und jeder aus Ring B und Ring C einen substituierten oder unsubstituierten, ein Stickstoffatom enthaltenden 5-gliedrigen oder 6-gliedrigen Ring darstellt; Z₁ beziehungsweise Z₂ den Ring B und den Ring C bildende Atomgruppen darstellen und jeder der Stickstoff-haltigen heterocyclischen Ringe wahlweise mit irgendeinem anderen Ring kondensiert ist; jedes aus A₁ und A₂ eine substituierte oder unsubstituierte Alkylgruppe, eine substituierte oder unsubstituierte Arylgruppe, eine substituierte oder unsubstituierte Alkylcarbonylgruppe, eine substituierte oder unsubstituierte Arylcarbonylgruppe, eine substituierte oder unsubstituierte Alkenylgruppe, einen substituierten oder unsubstituierten heterocyclischen Ringrest oder eine substituierte oder unsubstituierte Alkyloxycarbonylgruppe darstellt; jedes aus B₁ und B₂ eine substituierte oder unsubstituierte Alkylengruppe, eine substituierte oder unsubstituierte Alkenylengruppe oder eine substituierte oder unsubstituierte Arylengruppe darstellt; W -CH₂- oder-SO₂- darstellt; und n eine ganze Zahl von 0 oder 1 darstellt.

11. Optisches Aufzeichnungsmedium gemäß irgendeinem der Ansprüche 8 bis 10, wobei die Aufzeichnungsschicht ferner einen durch die folgende Strukturformel (4) ausgedrückten Cyaninfarbstoff umfasst: 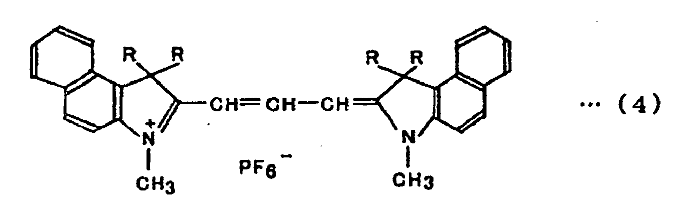 wobei jedes R -CH₃ oder eine Benzylgruppe darstellt, und mindestens zwei R Benzylgruppen sind.

12. Aufzeichnungs- und Wiedergabeverfahren eines optischen Aufzeichnungsmediums, umfassend:
Bestrahlen des optischen Aufzeichnungsmediums wie in irgendeinem der Ansprüche 8 bis 11 definiert mit einem Licht, das eine Aufzeichnungswellenlänge von 580 nm bis 720 nm aufweist, von der Seite des Substrats des optischen Aufzeichnungsmediums aus, um entweder Aufzeichnung oder Wiedergabe von Signalinformation durchzuführen.

## Revendications

1. Composé de squarylium formant un complexe métallique,
dans lequel le composé de squarylium est exprimé par la formule générale (I) ci-dessous : 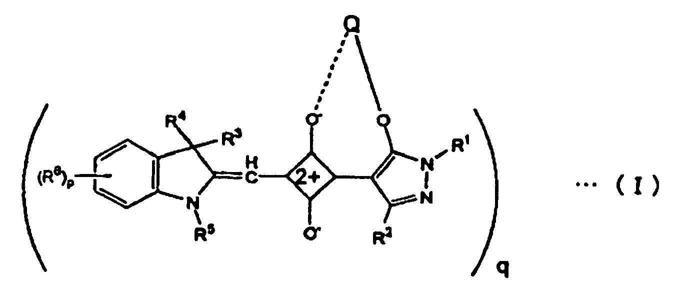 dans laquelle R¹ est un groupe phényle et R² est un groupe trifluorométhyle ou un groupe alkyle présentant une chaîne ramifiée ; Q représente un atome de métal présentant une aptitude à la coordination ; q représente 2 ou 3 ; R³ représente un groupe aralkyle substitué ou non substitué ou un groupe aryle substitué ou non substitué ; R⁴ représente un groupe alkyle substitué ou non substitué, un groupe aralkyle substitué ou non substitué, ou un groupe aryle substitué ou non substitué ; R⁵ représente un atome d'hydrogène, un groupe alkyle substitué ou non substitué, un groupe aralkyle substitué ou non substitué ou un groupe aryle substitué ou non substitué ; R⁶ représente un atome d'halogène, un groupe alkyle substitué ou non substitué, un groupe aralkyle substitué ou non substitué, un groupe aryle substitué ou non substitué, un groupe nitro, un groupe cyano ou un groupe alcoxy substitué ou non substitué ; p représente tout nombre entier compris entre 0 et 4 ; et les R⁶ sont identiques ou différents l'un de l'autre et deux R⁶ quelconques adjacents forment éventuellement un cycle aromatique substitué ou non substitué avec deux atomes de carbone adjacents lorsque p est égal à 2, 3 ou 4.

2. Composé de squarylium selon la revendication 1, dans lequel Q est l'aluminium.

3. Composé de squarylium selon l'une quelconque des revendications 1 et 2, dans lequel R² est un groupe isopropyle.

4. Composé de squarylium selon l'une quelconque des revendications 1 à 3, dans lequel R³ et R⁴ sont identiques ou différents et chacun de R³ et R⁴ est un groupe benzyle substitué ou non substitué ou un groupe phényle substitué ou non substitué.

5. Composé de squarylium selon la revendication 4, dans lequel chacun de R³ et R⁴ est un groupe benzyle.

6. Composé de squarylium selon la revendication 4, dans lequel R³ est un groupe phényle et R⁴ est un groupe benzyle.

7. Composé de squarylium selon l'une quelconque des revendications 1 à 6, dans lequel R⁵ est un groupe benzyle ou un groupe éthyle.

8. Support d'enregistrement optique comprenant :
un substrat ; et
une couche d'enregistrement disposée sur le substrat, dans lequel la couche d'enregistrement comprend au moins le composé de squarylium formant un complexe métallique comme défini dans l'une quelconque des revendications 1 à 7.

9. Support d'enregistrement optique selon la revendication 8, dans lequel la couche d'enregistrement comprend de plus un composé de squarylium formant un complexe métallique, dans lequel le composé de squarylium est exprimé par la formule générale (II) ci-dessous : 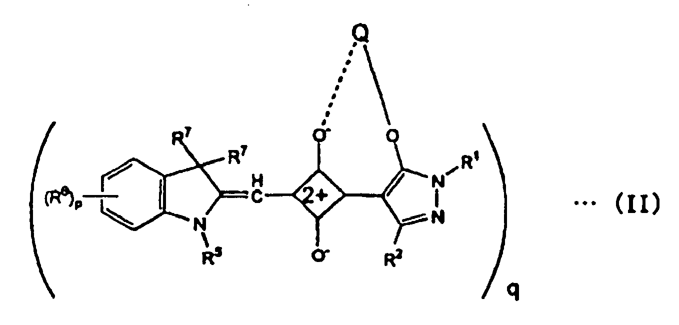 dans laquelle R¹ et R² sont identiques ou différents l'un de l'autre et chacun de R¹ et R² représente un groupe alkyle substitué ou non substitué, un groupe aralkyle substitué ou non substitué, un groupe aryle substitué ou non substitué ou un groupe hétérocyclique substitué ou non substitué ; Q représente un atome de métal présentant une aptitude à la coordination ; q représente 2 ou 3 ; R⁷ représente un groupe alkyle substitué ou non substitué, ou deux R⁷ sont éventuellement liés l'un à l'autre pour former soit un cycle hydrocarboné alicyclique, soit un cycle hétérocyclique ; R⁵ représente un atome d'hydrogène, un groupe alkyle substitué ou non substitué, un groupe aralkyle substitué ou non substitué ou un groupe aryle substitué ou non substitué ; R⁶ représente un atome d'halogène, un groupe alkyle substitué ou non substitué, un groupe aralkyle substitué ou non substitué, un groupe aryle substitué ou non substitué, un groupe nitro, un groupe cyano ou un groupe alcoxy substitué ou non substitué ; p représente tout nombre entier compris entre 0 et 4 ; et les R⁶ sont identiques ou différents l'un de l'autre et deux R⁶ quelconques adjacents forment éventuellement un cycle aromatique substitué ou non substitué avec deux atomes de carbone adjacents lorsque p est égal à 2, 3 ou 4.

10. Support d'enregistrement optique selon la revendication 8 ou 9, dans lequel la couche d'enregistrement comprend de plus un chélate de formazan et de métal formant un complexe métallique et un composé de formazan exprimé par la formule générale soit (III) soit (IV) ci-dessous : 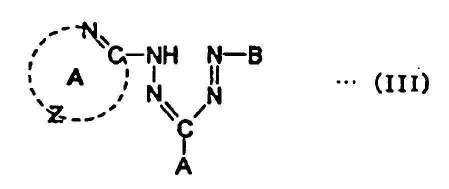 dans laquelle le cycle A représente un cycle à 5 éléments ou 6 éléments substitué ou non substitué contenant un atome d'azote ; Z représente un groupe atomique formant le cycle A dans lequel le cycle hétérocyclique contenant de l'azote est éventuellement condensé avec certains autres cycles ; A représente un groupe alkyle substitué ou non substitué, un groupe aryle substitué ou non substitué, un groupe alkylcarbonyle substitué ou non substitué, un groupe arylcarbonyle substitué ou non substitué, un groupe alcényle substitué ou non substitué, un résidu de cycle hétérocyclique substitué ou non substitué ou un groupe alkyloxycarbonyle substitué ou non substitué ; et B représente un groupe alkyle substitué ou non substitué, un groupe alcényle substitué ou non substitué ou un groupe aryle substitué ou non substitué, 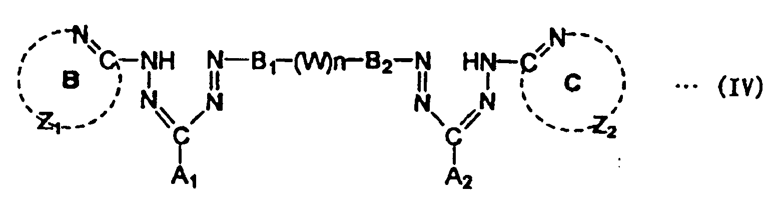 dans laquelle le cycle B et le cycle C sont identiques ou différents l'un de l'autre et chacun du cycle B et du cycle C représente un cycle à 5 éléments ou 6 éléments substitué ou non substitué contenant un atome d'azote ; Z₁ et Z₂ représentent respectivement des groupes atomiques formant le cycle B et le cycle C, et chacun des cycles hétérocycliques contenant de l'azote est éventuellement condensé avec certains autres cycles ; chacun de A₁ et A₂ représente un groupe alkyle substitué ou non substitué, un groupe aryle substitué ou non substitué, un groupe alkylcarbonyle substitué ou non substitué, un groupe arylcarbonyle substitué ou non substitué, un groupe alcényle substitué ou non substitué, un résidu de cycle hétérocyclique substitué ou non substitué ou un groupe alkyloxycarbonyle substitué ou non substitué ; chacun de B₁ et B₂ représente un groupe alkylène substitué ou non substitué, un groupe alcénylène substitué ou non substitué ou un groupe arylène substitué ou non substitué ; W représente -CH₂- ou -SO₂- et n représente un nombre entier égal à 0 ou à 1.

11. Support d'enregistrement optique selon l'une quelconque des revendications 8 à 10, dans lequel la couche d'enregistrement comprend de plus un colorant de cyanine étant exprimé par la formule de structure (4) suivante : 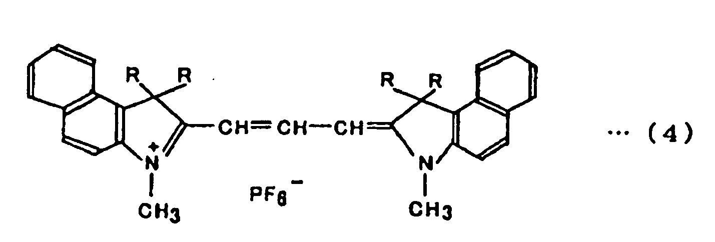 dans laquelle chacun des R représente -CH₃ ou un groupe benzyle et au moins deux R sont des groupes benzyle.

12. Procédé d'enregistrement et de reproduction d'un support d'enregistrement optique comprenant :
l'irradiation du support d'enregistrement optique selon l'une quelconque des revendications 8 à 11 avec une lumière présentant une longueur d'onde d'enregistrement de 580 nm à 720 nm à partir du côté du substrat du support d'enregistrement optique afin de réaliser soit un enregistrement, soit une reproduction d'information de signal.
